# EUROPEAN PATENT APPLICATION

(11) **EP 2 415 739 A1**
(43) Date of publication of application: **08.02.2012**
(21) Application number: 10758764.4
(22) Date of filing: 31.03.2010
(51) Int. Cl.: C07C 6/04, B01J 23/10, B01J 23/30, C07C 11/06, C07B 61/00, C07C 5/25

(54) **PROCESS FOR PRODUCING OLEFIN**

(30) Priority: 01.04.2009 JP 2009089008; 28.04.2009 JP 2009109346; 02.09.2009 JP 2009202892
(71) Applicant: Mitsui Chemicals, Inc., Tokyo 105-7117 (JP)
(72) Inventor: MIYAZOE, Satoru, Sodegaura-shi Chiba 299-0265 (JP); IKENAGA, Hirokazu, Sodegaura-shi Chiba 299-0265 (JP); KOTANI, Makoto, Sodegaura-shi Chiba 299-0265 (JP)
(74) Representative: HOFFMANN EITLE
(86) International application number: PCT/JP2010/055792
(87) International publication number: WO 2010/113993

(57) **Abstract**

[Object] It is an object of the present invention to provide a method for manufacturing an olefin, wherein side reactions of a metathesis reaction are suppressed and the selectivity of a desired product is increased by facilitating double bond isomerization of raw material olefins. Furthermore, it is an object to provide a method for manufacturing an olefin, wherein a desired product can be obtained efficiently at a high productivity by maintaining the activity of a metathesis catalyst for a long term and suppressing deterioration of performance of a catalyst (isomerization catalyst) for facilitating double bond isomerization of the raw material olefins.

[Solution] A method for manufacturing an olefin according to the present invention is a method for manufacturing an olefin through reaction between the same type of or different types of raw material olefins to obtain an olefin having a structure different from the structure of the raw material olefins, the method including using a catalyst containing at least one type of metal element selected from the group consisting of tungsten, molybdenum, and rhenium together with other specific catalysts.

## Description

### [Technical Field]

The present invention relates to a method for manufacturing an olefin through a metathesis reaction between the same type of or different types of olefins.

### [Background Art]

The metathesis reaction, in which the same type of or different types of olefins react with each other to produce an olefin having a different structure, brings about significant advantages because, for example, ethylene, propylene, butene, and the like produced from a naphtha cracker at certain proportions are converted to each other and, thereby, it is possible to respond changes in structure of demand for olefins.

Most of all, regarding the metathesis reaction by using an internal olefin as a raw material, it is known that a combination of a metathesis catalyst and a catalyst for isomerizing a double bond of an olefin is used.
For example, in a process for producing propylene from ethylene and n-butene and a process for producing neohexene from 2,4,4-trimethylpentene and ethylene, magnesium oxide having activity in double bond isomerization is used in combination with the metathesis catalyst (Non-Patent Literature 1).

It is indicated that the above-described double bond isomerization catalyst typified by magnesium oxide contributes to not only isomerization, but also an improvement of metathesis activity.

For example, Non-Patent Literature 2 describes that in the metathesis reaction between propylenes, the conversion is increased to 44% or more by using a metathesis catalyst, in which tungsten oxide is supported with a silica carrier, together with magnesium oxide whereas the conversion is 4% in the case where the metathesis catalyst is used alone. Patent Literature 1 discloses that in a method for manufacturing propylene through the metathesis reaction between ethylene and 2-butene, the conversion is increased by using a mixed catalyst composed of a metathesis catalyst, in which tungsten oxide is supported on a silica carrier, and a magnesium oxide catalyst.

Furthermore, Patent Literature 2 discloses that in a method for manufacturing propylene through the metathesis reaction between ethylene and n-butene, the metathesis reaction proceeds at an industrially sufficient reaction rate even in a low-temperature range by allowing a small amount of hydrogen to present together with a mixed catalyst composed of a metathesis catalyst and a co-catalyst.

Moreover, regarding the metathesis reaction, in which an internal olefin is used as a raw material, as is cited from Non-Patent Literature 1, it is estimated that the isomerization activity has an influence on the productivity of a desired product.

For example, propylene is not generated in the metathesis reaction between 1-butene and ethylene and neohexene is not generated in the metathesis reaction between 2,4,4-trimethyl-1-pentene and ethylene. Therefore, it is estimated that the isomerization activity has an influence on the productivity of a desired product.

Non-Patent Literature 3 mentions production of 2-pentene through metathesis of propylene, which is a primary product, and 1-butene, production of 2-pentene through metathesis of 2-butene and 1-butene, and production of 3-hexene through metathesis of 1-butene with each other as main side reactions in the metathesis reaction between ethylene and n-butene. Therefore, it is indicated that the presence of 1-butene leads to a reduction in selectivity.

In addition, Patent Literature 3 discloses that regarding the metathesis reaction of propylene, the activity is enhanced in the case where the metathesis catalyst supported with a carrier, e.g., silica, further contains a rare-earth metal oxide whereas the isomerization activity is reduced.

As described above, regarding the methods for manufacturing an olefin, the method including a metathesis reaction step, various inventions have been made. However, the olefin yields of these manufacturing methods do not completely satisfy the requirements of persons skilled in the related art, and a manufacturing method further excellent in selectivity of desired products has been desired.

On the other hand, regarding manufacturing methods, in which compounds formed from alkaline earth metal elements and rare-earth metal elements are used as catalysts, a method for synthesizing a hydrocarbon primarily containing ethane and ethylene by reacting methane and oxygen at high temperatures in the presence of these composite oxide catalysts (Patent Literature 4), a Michael addition reaction by using a composite oxide of magnesium and lanthanum as a catalyst (Non-Patent Literature 4), and the like are known.

However, nothing is known about usage of compounds formed from alkaline earth metal elements and rare-earth metal elements for the metathesis reaction of olefins and an effect thereof on the reaction.

Furthermore, a cyanoethylation reaction of ethanol by using a calcined product of hydrotalcite containing magnesium, aluminum, and a rare-earth metal (yttrium or lanthanum) as components is known as a reaction, in which a metal oxide containing three components including an alkaline earth metal element, aluminum, and a rare-earth metal element is used as a catalyst (Non-Patent Literature 5).

However, nothing is known about usage of a metal oxide containing three components including an alkaline earth metal element, aluminum, and a rare-earth metal element for the isomerization catalyst in the metathesis reaction of olefins and an effect on the metathesis reaction.

Moreover, regarding metal oxides containing three components including an alkaline earth metal element, aluminum, and a rare-earth metal element, many ceramic materials have been reported, although most of them have merely a small content of one of the above-described three components. As for a metal oxide having relatively high individual contents of the above-described three components, Patent Literature 5 discloses corrosion-resistant ceramic containing magnesium, aluminum, and yttrium as components.

However, production is conducted through sintering at a high temperature of 1,600°C, and a specific surface area useful as a catalyst cannot be expected.

In addition, compounds having a layered double hydroxide (LDH) structure have been reported in many literatures, e.g., Non-Patent Literature 6. In this regard, as for the layered double hydroxide containing three components including an alkaline earth metal element, aluminum, and a rare-earth metal element, Non-Patent Literature 5, cited above, discloses a compound having a "hydrotalcite" structure, which is one type of LDH, and containing the above-described three components.

However, nothing is known about usefulness in usage as a catalyst precursor for the metathesis reaction and a composition range useful for the above-described catalyst precursor.

Furthermore, as is described in Patent Literature 6, it is known to a person skilled in the related art that the metathesis catalyst is influenced sensitively by a catalyst poison contained in raw material olefins supplied to a reactor. Examples of catalyst poisons include hetero-atom-containing compounds, e.g., water, alcohols, ethers, ketones, aldehydes, acids, acid derivatives, amines, nitriles, and thiols, acetylene, and dienes. The activity of the metathesis catalyst is reduced by such catalyst poisons, and the cycle time or the life of the metathesis catalyst is reduced significantly.

Various methods for dealing with such influences of catalyst poisons have been known (Patent Literature 6). For example, a method, in which acetylene and dienes are reduced by selectively hydrogenating raw material olefins, and a method, in which hetero-atom-containing compounds are reduced by treating raw material olefins through adsorption, are known. Moreover, Patent Literature 7 discloses that in the case where a catalyst containing alumina as a primary component is used together with the metathesis catalyst, the life of the metathesis reaction activity is extended significantly and, thereby, olefins can be produced over a practical term.

However, regarding the method for manufacturing an olefin by using the metathesis catalyst and the isomerization catalyst, nothing is known about a method for suppressing deterioration of performance of the isomerization catalyst.

### [Citation List]

### [Patent Literature]

[PTL 1] US Patent No. 4, 575, 575
[PTL 2] International Patent Publication WO 06/093058
[PTL 3] US Patent No. 3,883,606
[PTL 4] Japanese Unexamined Patent Application Publication No. 01-168626
[PTL 5] Japanese Unexamined Patent Application Publication No. 2002-362966
[PTL 6] International Patent Publication WO 06/089957
[PTL 7] Japanese Unexamined Patent Application

### Publication No. 05-103995

### [Non Patent Literature]

[NPL 1] J.C.Mol, "Industrial applications of olefin metathesis", Journal of Molecular Catalysis A: Chemical, Vol. 213, p. 39-45 (2004)
[NPL 2] ROBERT L. BANKS and SIMON G. KUKES, "NEW DEVELOPMENTS AND CONCEPTS IN ENHANCING ACTIVITIES OF HETEROGENEOUS METATHESIS CATALYSTS", Journal of Molecular Catalysis, Vol. 28, p. 117-131 (1985)
[NPL 3] Keisuke Ishii et al., "ABB Ru-masu Guro-baru Puropiren Seizou Gijutsu (ABB Lummus Global Propylene Production Technology)", PETROTECH, Vol. 26, p. 484-489 (2003)
[NPL 4] Bhaskar Veldurthy et al., "Magnesium-Lanthanum Mixed Metal Oxide: a Strong Solid Base for the Michael Addition Reaction", Advanced Synthesis and Catalysis, Vol. 347, p. 767-771 (2005)
[NPL 5] Emilian Angelescu et al., "Cyanoethylation of ethanol on Mg-Al hydrotalcites promoted by Y3+ and Lea3+", Catalysis Communications, Vol. 5, p. 647-651 (2004)
[NPL 6] F. Cavani et al., "Hydrotalcite-type Anionic Clays: Preparation, Properties and Application", Catalysis Today, Vol. 11, p. 173-301 (1991)

### [Summary of Invention]

### [Technical Problem]

It is an object of the present invention to provide a method for manufacturing an olefin, wherein side reactions of a metathesis reaction are suppressed and the selectivity of a desired product is increased by facilitating double bond isomerization of raw material olefins. Furthermore, it is also an object to provide a catalyst useful for the above-described manufacturing method and a precursor of the above-described catalyst. Moreover, it is an object to provide a method for manufacturing an olefin, wherein a desired product can be obtained efficiently at a high productivity by maintaining the activity of a metathesis catalyst for a long term and suppressing deterioration of performance of a catalyst (isomerization catalyst) for facilitating double bond isomerization of raw material olefins.

### [Solution to Problem]

The present inventors considered that enhancement of isomerization activity of an isomerization catalyst used together with a metathesis catalyst was necessary to obtain a desired product efficiently at a high productivity, in particular in the metathesis reaction, in which an internal olefin was used as a raw material, among metathesis reactions and made an intensive search for a catalyst having isomerization activity higher than that of a publicly known magnesium oxide catalyst. As a result, it was found that a metal oxide catalyst composed of an alkaline earth metal, e.g., magnesium, and a rare-earth metal, e.g., yttrium, exhibited excellent activity as an isomerization catalyst and, thereby, a first aspect of the invention has been completed. Furthermore, it was found that a metal oxide composed of an alkaline earth metal, e.g., magnesium, aluminum, and a rare-earth metal, e.g., yttrium, exhibited excellent activity as an isomerization catalyst and, thereby, a second aspect of the invention has been completed. Moreover, it was found that in the case where a catalyst containing alumina is used together with the metathesis catalyst and the isomerization catalyst, not only the activity of the metathesis catalyst was able to be maintained for a long term, but also deterioration of performance of the isomerization catalyst was able to be suppressed and, thereby, a third aspect of the invention has been completed.

That is, the present invention relates to the following items (1) to (27).

(1) A method for manufacturing an olefin through reaction between the same type of or different types of raw material olefins to obtain an olefin having a structure different from the structure of the raw material olefins, the method including using
   a catalyst containing at least one type of metal element selected from the group consisting of tungsten, molybdenum, and rhenium, and
   a metal oxide catalyst containing at least two types of metal elements including at least one type of metal element selected from alkaline earth metal elements and at least one type of metal element selected from rare earth elements.

(2) A method for manufacturing an olefin through reaction between the same type of or different types of raw material olefins to obtain an olefin having a structure different from the structure of the raw material olefins, the method including using
   a catalyst containing at least one type of metal element selected from the group consisting of tungsten, molybdenum, and rhenium, and
   a metal oxide catalyst containing three components including at least one type of metal element (component A) selected from alkaline earth metal elements, aluminum (component B), and at least one type of metal element (component C) selected from rare earth elements.

(3) A method for manufacturing an olefin through reaction between the same type of or different types of raw material olefins to obtain an olefin having a structure different from the structure of the raw material olefins, the method including using
   a catalyst (a) containing at least one type of metal element selected from the group consisting of tungsten, molybdenum, and rhenium,
   a catalyst (b) containing at least one type of metal element selected from alkaline earth metal elements and rare earth elements; and
   a catalyst (c) containing alumina.

(4) The method for manufacturing an olefin according to the above-described item (1) including the steps of
   obtaining an olefin from the same type or different types of olefins among raw material olefins including an olefin having the carbon number of 3 or more, the resulting olefin being different from the same type or the different types of olefins, in a reactor containing
   a metathesis catalyst, as a metathesis reaction step, and
   isomerizing an olefin to shift the position of a double bond within the molecule in a reactor which contains an isomerization catalyst and in which raw material olefins are present which are the same as or different from the above-described raw material olefins and include an olefin having the carbon number of 3 or more,
   wherein the above-described metathesis catalyst is a catalyst containing at least one type of metal element selected from the group consisting of tungsten, molybdenum, and rhenium, and the above-described isomerization catalyst is a metal oxide catalyst containing at least two types of metal elements including at least one type of metal element selected from alkaline earth metal elements and at least one type of metal element selected from rare earth elements.

(5) The method for manufacturing an olefin according to the above-described item (2) including the steps of
   obtaining an olefin from the same type or different types of olefins among raw material olefins including an olefin having the carbon number of 3 or more, the resulting olefin being different from the same type or the different types of olefins, in a reactor containing a metathesis catalyst, as a metathesis reaction step, and
   isomerizing an olefin to shift the position of a double bond within the molecule in a reactor which contains an isomerization catalyst and in which raw material olefins are present which are the same as or different from the above-described raw material olefins and include an olefin having the carbon number of 3 or more,
   wherein the above-described metathesis catalyst is
   a catalyst containing at least one type of metal element
   selected from the group consisting of tungsten, molybdenum, and rhenium, and
   the above-described isomerization catalyst is a metal oxide catalyst containing the following three components including
   at least one type of metal element (component A) selected from alkaline earth metal elements,
   aluminum (component B), and
   at least one type of metal element (component C) selected from rare earth elements.

(6) The method for manufacturing an olefin according to the above-described item (3) including the steps of
   obtaining an olefin from the same type or different types of olefins among raw material olefins including an olefin having the carbon number of 3 or more, the resulting olefin being different from the same type or the different types of olefins, in a reactor containing a metathesis catalyst, as a metathesis reaction step, and
   isomerizing an olefin to shift the position of a double bond within the molecule in a reactor which contains an isomerization catalyst and in which raw material olefins are present which are the same as or different from the above-described raw material olefins and include an olefin having the carbon number of 3 or more,
   wherein the above-described metathesis catalyst is a catalyst (a) containing at least one type of metal element selected from the group consisting of tungsten, molybdenum, and rhenium,
   the above-described isomerization catalyst is a metal oxide catalyst (b) containing at least one type of metal element selected from alkaline earth metal elements and rare earth elements, and
   a catalyst (c) containing alumina is used together with the above-described metathesis catalyst and/or the above-described isomerization catalyst.

(7) The method for manufacturing an olefin according to any one of the above-described items (4) to (6), **characterized in that** the above-described isomerization reaction step and the above-described metathesis reaction step are conducted in one reactor.

(8) The method for manufacturing an olefin according to any one of the above-described items (4) to (6), **characterized in that** the above-described isomerization reaction step and the above-described metathesis reaction step are conducted in separate reactors, and the olefin generated in the isomerization reaction step is used as at least a part of the raw material olefins for the metathesis reaction step.

(9) The method for manufacturing an olefin according to, any one of the above-described items (1) to (3), **characterized in that** hydrogen is also supplied together with the raw material olefins.

(10) The method for manufacturing an olefin according to any one of the abave-described items (4) to (8), **characterized in that** hydrogen is also supplied together with the raw material in at least one of the above-described isomerization reaction step and the above-described metathesis reaction step.

(11) The method for manufacturing an olefin according to any one of the above-described items (4) to (8), wherein the raw material olefines including an olefin having the carbon number of 3 or more contains n-butene.

(12) The method for manufacturing an olefin according to any one of the above-described items (1) to (11), wherein the above-described raw material olefins contain ethylene.

(13) The method for manufacturing an olefin according to any one of the above-described items (1) to (12), **characterized in that** the above-described metal element selected from the alkaline earth metals is magnesium or calcium.

(14) The method for manufacturing an olefin according to any one of the above-described items (1) to (13), **characterized in that** the above-described metal element selected from the rare earth elements is yttrium or lanthanum.

(15) The method for manufacturing an olefin according to the above-described item (3) or the item (6), **characterized in that** the above-described catalyst (c) is a catalyst having a specific surface area of 50 m²/g or more.

(16) The method for manufacturing an olefin according to the above-described item (3) or the item (6), **characterized in that** the above-described catalyst (a), the above-described catalyst (b), and the above-described catalyst (c) are formed bodies made independently, and the above-described individual formed bodies are present together in one reactor.

(17) The method for manufacturing an olefin according to the above-described item (3) or the item (6), **characterized in that** the catalyst (a) is a formed body (i) made independently, the catalyst (b) and the catalyst (c) constitute a formed body (ii) by being mixed with each other, and the above-described formed body (i) and the above-described formed body (ii) are present together in one reactor.

(18) The method for manufacturing an olefin according to the above-described item (2) or the item (5), wherein the content of the component A in the above-described isomerization catalyst is 80% or less on a mole fraction basis, where a total of metal elements of the components A, B, and C is assumed to be 100%.

(19) The method for manufacturing an olefin according to the above-described item (2) or the item (5), wherein the content of the component B in the above-described isomerization catalyst is 50% or less on a mole fraction basis, where a total of metal elements of the components A, B, and C is assumed to be 100%.

(20) The method for manufacturing an olefin according to the above-described items (2) or the item (5), wherein the content of the component C in the abode-described isomerization catalyst is 5% or more on a mole fraction basis, where a total of metal elements of the components A, B, and C is assumed to be 100%.

(21) An isomerization catalyst for producing an olefin comprising a metal oxide containing three components including at least one type of metal element (component A) selected from alkaline earth metal elements, aluminum (component B), and at least one type of metal element (component C) selected from rare earth elements.

(22) The isomerization catalyst according to the above-described item (21), wherein the content of the component A is 80% or less on a mole fraction basis, where a total of metal elements of the components A, B, and C is assumed to be 100%.

(23) The isomerization catalyst according to the above-described item (21) or the item (22), wherein the content of the component B is 50% or less on a mole fraction basis, where a total of metal elements of the components A, B, and C is assumed to be 100%.

(24) The isomerization catalyst according to any one of the above-described items (21) to (23), wherein the content of the component C is 5% or more on a mole fraction basis, where a total of metal elements of the components A, B, and C is assumed to be 100%.

(25) The isomerization catalyst according to any one of the above-described items (21) to (24), wherein the specific surface area is 10 m²/g or more.

(26) The isomerization catalyst according to any one of the above-described items (21) to (25), **characterized in that**
   the above-described isomerization catalyst is produced by calcining a catalyst precursor,
   the above-described catalyst precursor contains
   at least one type of metal element (component A) selected from alkaline earth metal elements,
   aluminum (component B), and
   at least one type of metal element (component C) selected from rare earth elements,
   wherein at least a part thereof has a layered double hydroxide (LDH) structure.

(27) A catalyst precursor of an isomerization catalyst for producing an olefin, the catalyst precursor characterized by containing
   at least one type of metal element (component A) selected from alkaline earth metal elements,
   aluminum (component B), and
   at least one type of metal element (component C) selected from rare earth elements,
   wherein 30% to 80% of component A, 9% to 50% of component B, and 5% to 50% of component C are contained on a mole fraction basis, where a total of metal elements of the components A, B, and C is assumed to be 100%, and at least a part thereof has a layered double hydroxide (LDH) structure.

### [Advantageous Effects of Invention]

Regarding the manufacturing method according to the present invention, side reactions are suppressed and the selectivity of a desired product is increased by facilitating isomerization of a double bond of olefins. Furthermore, the isomerization catalyst used in the manufacturing method according to the present invention can select the ratio of contents of the alkaline earth metal and the rare-earth metal or contents of the alkaline earth metal, aluminum, and the rare-earth metal over a wide range while excellent isomerization performance is maintained. Therefore, the usage of the rare-earth metal can be reduced and excellent industrial economy is exhibited.

Moreover, regarding the manufacturing method according to the present invention, the catalyst containing aluminum is used together with the metathesis catalyst and the isomerization catalyst and, thereby, not only the activity of the metathesis catalyst is maintained for a long term, but also deterioration of performance of the isomerization catalyst is suppressed, so that the term, in which a desired product is obtained at a high selectivity, can be extended. In this regard, particularly in the case where the catalyst containing aluminum is used together with the isomerization catalyst having excellent activity, a desired product can be obtained at a very high selectivity for a long term continuously. In addition, in the case where the isomerization catalyst contains the rare-earth metal, the usage of the rare-earth metal can be reduced and excellent industrial economy is exhibited.

### [Brief Description of Drawings]

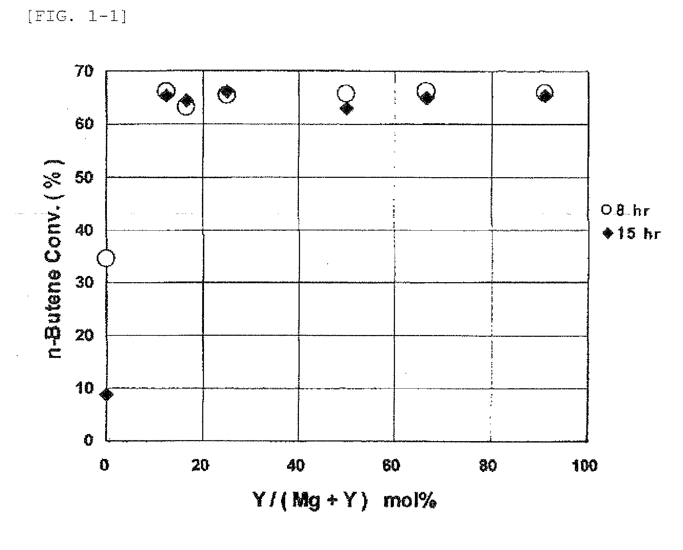

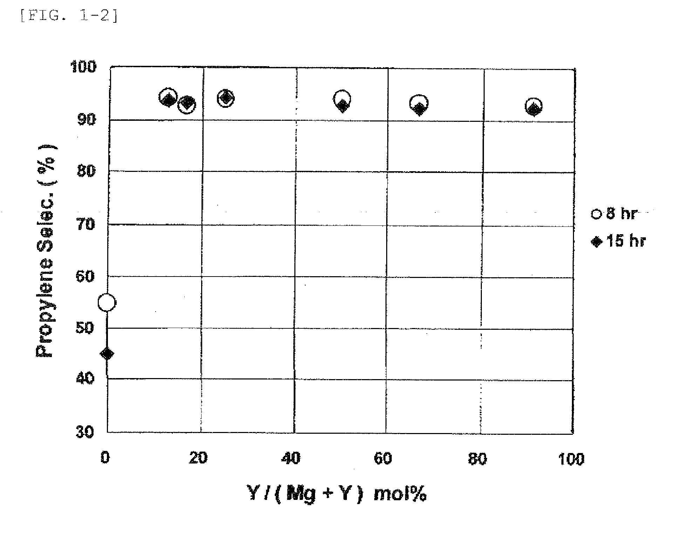

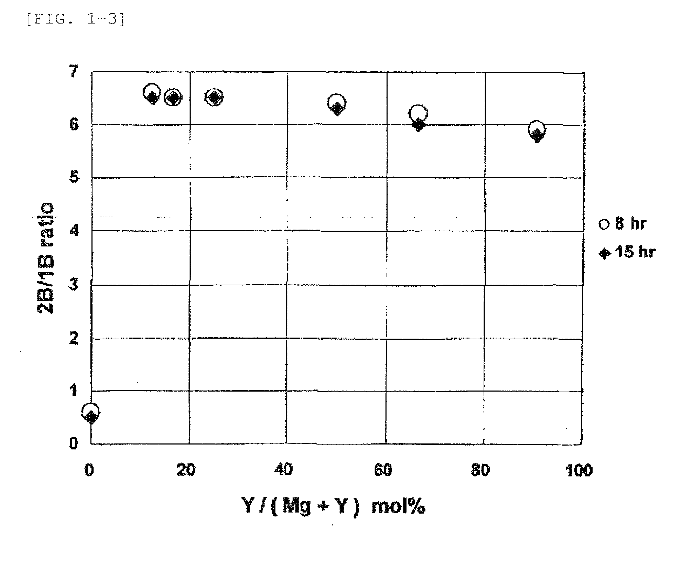

[Fig. 1-1] Fig. 1-1 is a graph showing the relationship between the butene conversion and the composition of the isomerization catalyst containing Mg and Y in Examples 1-1 to 1-6 and Comparative example 1-1.
[Fig. 1-2] Fig. 1-2 is a graph showing the relationship between the propylene selectivity and the composition of the isomerization catalyst containing Mg and Y in Examples 1-1 to 1-6 and Comparative example 1-1.
[Fig. 1-3] Fig. 1-3 is a graph showing the relationship between the 2-butene/1-butene ratio and the composition of the isomerization catalyst containing Mg and Y in Examples I-1 to 1-6 and Comparative example 1-1.
[Fig. 2-1] Fig. 2-1 is a graph showing the butene conversions in Examples 2-1 to 2-11 and Comparative example 2-1.
[Fig. 2-2] Fig. 2-2 is a graph showing the propylene selectivities in Examples 2-1 to 2-11 and Comparative example 2-1.

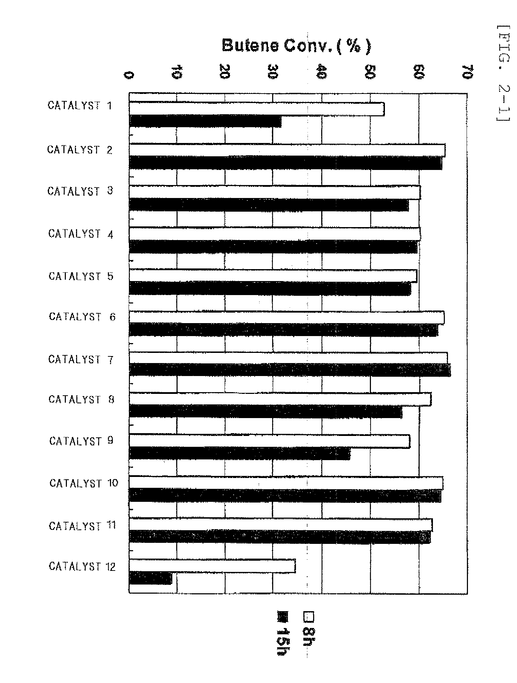

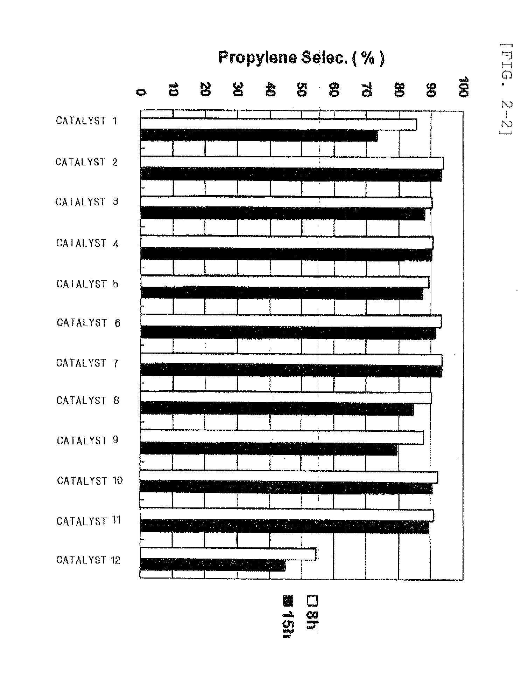

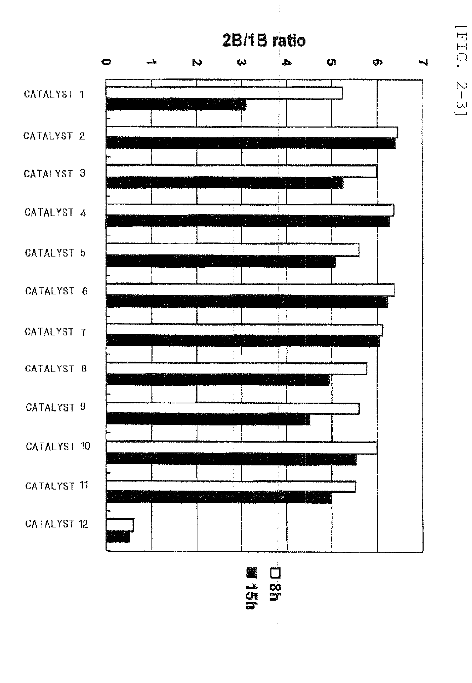

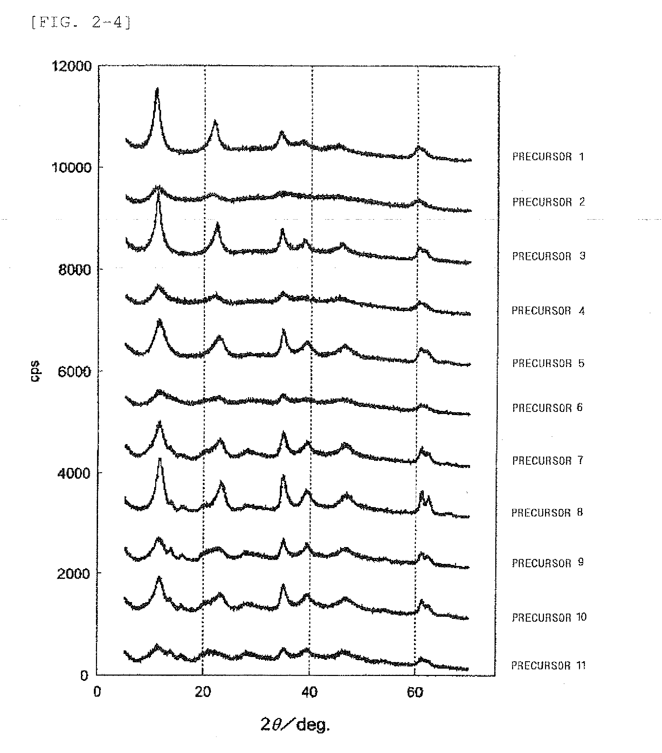

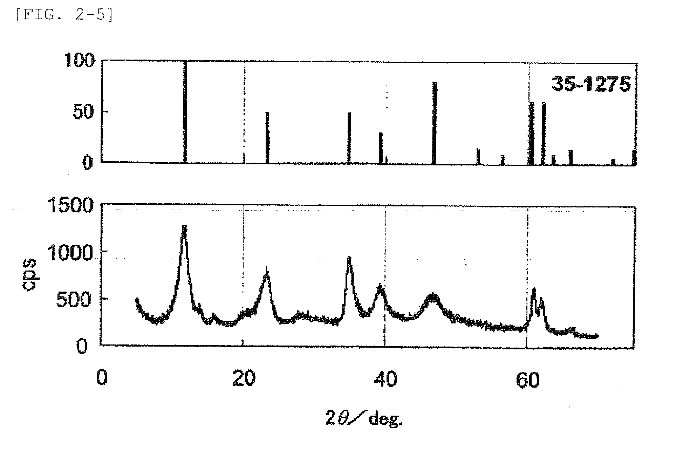

[Fig. 2-3] Fig. 2-3 is a graph showing the 2-butene/l-butene ratios in Examples 2-1 to 2-11 and Comparative example 2-1.
[Fig. 2-4] Fig. 2-4 shows X-ray diffraction patterns of Catalyst precursors 1 to 11.
[Fig. 2-5] Fig. 2-5 is a diagram for comparing the X-ray diffraction pattern of Catalyst precursor 8 with JCPDS Card No. 35-1275 Mg₂Al(OH)₇.

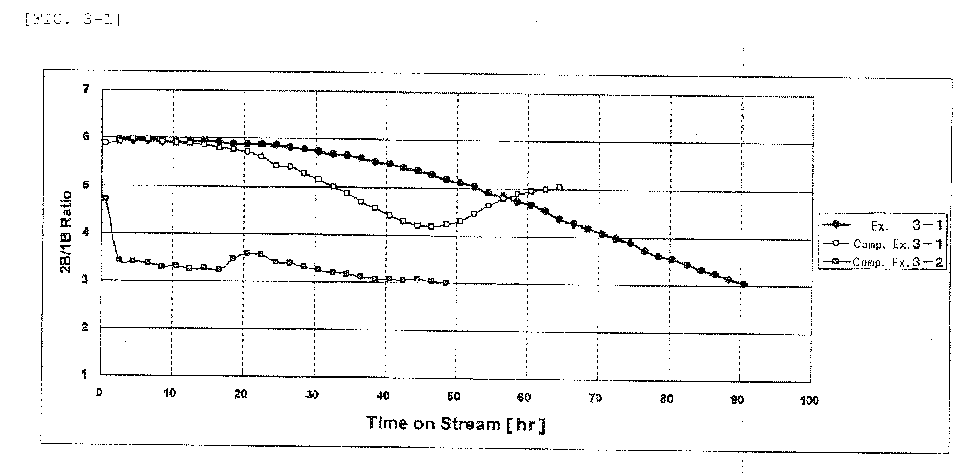

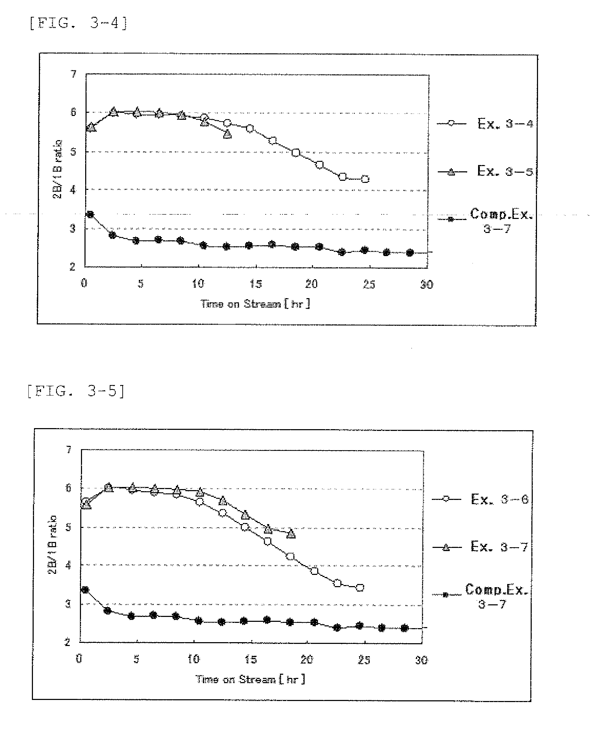

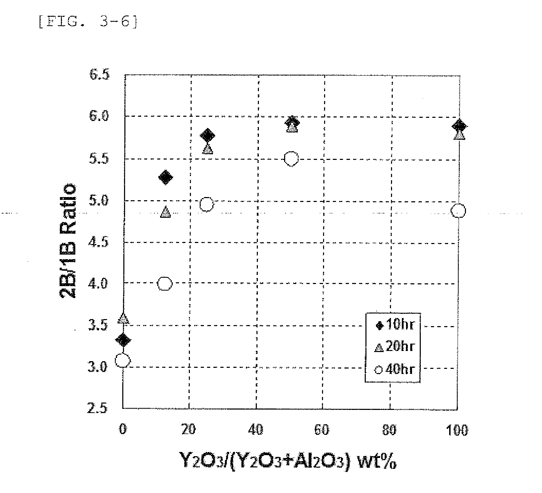

[Fig. 3-1] Fig. 3-1 is a graph showing the progression of 2-butene/1-butene ratios with time in Example 3-1, Comparative example 3-1, and Comparative example 3-2.

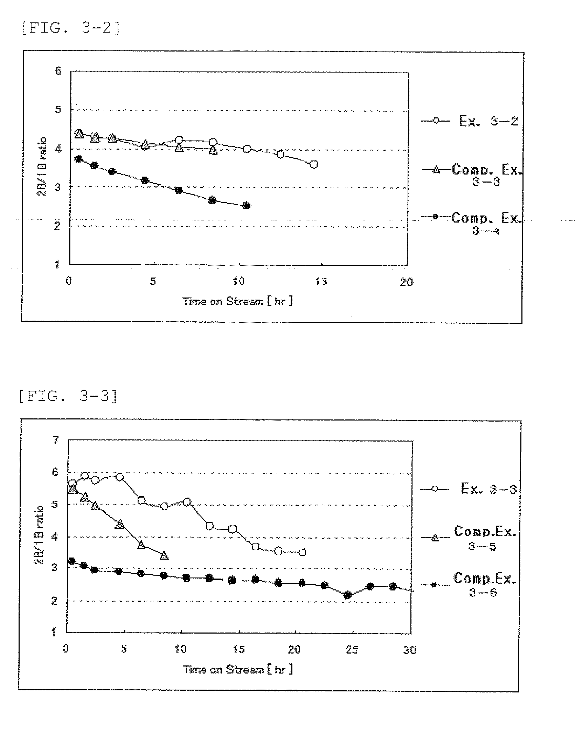

[Fig. 3-2] Fig. 3-2 is a graph showing the progression of 2-butene/1-butene ratios with time in Example 3-2, Comparative example 3-3 and Comparative example 3-4.
[Fig. 3-3] Fig. 3-3 is a graph showing the progression of 2-butene/1-butene ratios with time in Example 3-3, Comparative example 3-5 and Comparative example 3-6.
[Fig. 3-4] Fig. 3-4 is a graph showing the progression of 2-butene/1-butene ratios with time in Example 3-4, Example and Comparative example 3-7.
[Fig. 3-5] Fig. 3-5 is a graph showing the progression of 2-butene/1-butene ratios with time in Example 3-6, Example 3-7 and Comparative example 3-7.
[Fig. 3-6] Fig. 3-6 is a graph showing the relationships between 2-butene/1-butene ratio and the catalyst blend ratio after 10, 20 and 40 hours from the initiation of the reactions in Example 3-1, Examples 3-8 to 3-9 and Comparative examples 3-1 to 3-2.

### [Description of Embodiments]

A method for manufacturing an olefin according to the present invention is a method for manufacturing an olefin through reaction between the same type of or different types of raw material olefins to obtain an olefin having a structure different from the structure of the raw material olefins (a method for manufacturing an olefin through reaction between the same type of or different types of olefins to obtain an olefin having a structure different from the structure of the same type of or different types of olefins), the method including using a catalyst containing at least one type of metal element selected from the group consisting of tungsten, molybdenum, and rhenium together with other specific catalysts.

A first aspect of the method for manufacturing an olefin according to the present invention comprises using a catalyst containing at least one type of metal element selected from the group consisting of tungsten, molybdenum, and rhenium and a metal oxide catalyst containing at least two types of metal elements including at least one type of metal element selected from alkaline earth metal elements and at least one type of metal element selected from rare earth elements.

A second aspect of the method for manufacturing an olefin according to the present invention comprises using a catalyst containing at least one type of metal element selected from the group consisting of tungsten, molybdenum, and rhenium and a metal oxide catalyst containing three components including at least one type of metal element (component A) selected from alkaline earth metal elements, aluminum (component B), and at least one type of metal element (component C) selected from rare earth elements.

A third aspect of the method for manufacturing an olefin according to the present invention comprises a catalyst (a) containing at least one type of metal element selected from the group consisting of tungsten, molybdenum, and rhenium, a catalyst (b) containing at least one type of metal element selected from alkaline earth metal elements and rare earth elements, and a catalyst (c) containing alumina.

It is preferable that the first to the third aspects of the method for manufacturing an olefin according to the present invention are applied to a method for manufacturing an olefin including the steps of obtaining an olefin from the same type or different types of olefins among raw material olefins including an olefin having the carbon number of 3 or more, the resulting olefin being different from the same type or the different types of olefins, in a reactor containing a metathesis catalyst, as a metathesis reaction step and isomerizing an olefin to shift the position of a double bond within the molecule in a reactor which contains an isomerization catalyst and in which raw material olefins are present which are the same as or different from the above-described raw material olefins and include an olefin having the carbon number of 3 or more.

The first to the third aspects of the method for manufacturing an olefin according to the present invention will be described below in detail.

The first aspect of the manufacturing method according to the present invention is a method for manufacturing an olefin, the method including two reaction treatments of the metathesis reaction and the isomerization reaction of the double bond and being **characterized in that**
the metathesis catalyst contains at least one type of metal element selected from the group consisting of tungsten, molybdenum, and rhenium and
the isomerization catalyst is a metal oxide catalyst containing at least two types of metal elements including at least one type of metal element selected from alkaline earth metal elements and at least one type of metal element selected from rare earth elements.

In the first aspect of the manufacturing method according to the present invention, the metathesis catalyst is not specifically limited insofar as the metathesis catalyst contains at least one type of metal element selected from the group consisting of tungsten, molybdenum, and rhenium. The structure of the catalyst containing tungsten, molybdenum, or rhenium is not limited. For example, oxides, sulfides, hydroxides, and the like can be used. Most of all, oxides, e.g., WO₃, MoO₃, Re₂O₇, are preferable, and WO₃ is further preferable. These metal elements may be fixed to a carrier, e.g., silica, alumina, or aluminum phosphate. In many cases, a tungsten oxide catalyst supported with silica or a rhenium catalyst supported with alumina is used. The catalysts, in which a reaction-facilitating component, e.g., alkali metals, alkaline earth metals, silicon, zinc, zirconium, and chromium, are further supported together, can also be used.

In the first aspect of the manufacturing method according to the present invention, the isomerization catalyst is a metal oxide catalyst containing at least two types of metal elements including at least one type of metal element selected from alkaline earth metal elements and at least one type of metal element selected from rare earth elements. It is necessary that at least a part of individual metal elements form a mixed state which cannot be separated physically, although formation of a completely single composite oxide phase is not necessary. Even when diffraction peaks attributed to the alkaline earth metal oxide and the rare-earth metal oxide are present together in the X-ray diffraction measurement, it is possible to use as the isomerization catalyst in the first aspect according to the present invention, insofar as the oxides are not separated physically.

In the first aspect of the manufacturing method according to the present invention, the metal oxide catalyst is not necessarily formed from only an oxide of the metal element, and a hydroxide and a carbonate may be contained. Furthermore, as long as a metal oxide catalyst is ready to use at the time of production of the olefin, the configuration before the use is not specifically limited. Organic acid salts, alkoxides, and the like may be employed insofar as they can be converted to a metal oxide catalyst through a calcining treatment in an air atmosphere. For example, organic acid salt coprecipitates of Mg and Y can be converted to the above-described metal oxide catalyst by putting the coprecipitates as-is into a reactor and conducting the calcining treatment.

In the first aspect of the manufacturing method according to the present invention, the metal element selected from the alkaline earth metals is not specifically limited insofar as the metal element is selected from group 2 of the periodic table. Magnesium and calcium are preferable from the viewpoint of industrial availability and price, and magnesium is further preferable.

In the first aspect of the manufacturing method according to the present invention, the metal element selected from the rare earth elements is not specifically limited insofar as the metal element is included in a group composed of Lanthanoids having atomic numbers of 57 to 71, scandium, and yttrium. Yttrium, or Lanthanoids having atomic numbers of 57 to 63 are preferable from the viewpoint of industrial availability and price, and yttrium or Lanthanum is further preferable.

In the first aspect of the manufacturing method according to the present invention, the ratio of amount of alkaline earth metals to rare-earth metals contained in the isomerization catalyst is not specifically limited likewise. However, the molar ratio of alkaline earth metals/rare-earth metals is preferably 20 to 0.1, and further preferably 10 to 0.5.

In the first aspect of the manufacturing method according to the present invention, the method for manufacturing the isomerization catalyst is not specifically limited, and any method of, for example, an impregnation method, an incipient wetness method, a spraying method, a coprecipitation method, a sol-gel method, and a thermal decomposition method (from chlorides, organometals, organic acid salts, and the like) may be employed, insofar as it is the method for preparing a solid catalyst. Among the above-described preparation methods, the coprecipitation method, the sol-gel method, or the thermal decomposition method (from chlorides, organometals, organic acid salts, and the like) is preferable because a desirable mixed state of the alkaline earth metal and the rare-earth metal can be formed easily. The form of the isomerization catalyst is not specifically limited. The isomerization catalyst may be in the shape of a powder or granules, be formed into various shapes, or be fixed on a surface of a support, e.g., a honeycomb, a mesh sheet, and a filter. Furthermore, the catalyst can also be used while being supported on a surface of a catalyst carrier.

According to the above-described first aspect of the manufacturing method of the present invention, double bond isomerization of the raw material olefins can be facilitated and side reactions of the metathesis reaction can be suppressed. As a result, a desired product can be obtained from the raw material olefins at a high selectivity.

The second aspect of the manufacturing method according to the present invention is a method for manufacturing an olefin, the method including two reaction treatments of the metathesis reaction and the double bond isomerization reaction and being **characterized in that**
the metathesis catalyst contains at least one type of metal element selected from the group consisting of tungsten, molybdenum, and rhenium and
the isomerization catalyst is a metal oxide catalyst containing three components including at least one type of metal element (component A) selected from alkaline earth metal elements, aluminum (component B), and at least one type of metal element (component C) selected from rare earth elements.

The metathesis catalyst in the second aspect of the manufacturing method according to the present invention is the same metathesis catalyst as that in the above-described first aspect.

The isomerization catalyst used in the second aspect of the manufacturing method according to the present invention is a metal oxide catalyst containing three components including at least one type of metal element (component A) selected from alkaline earth metal elements, aluminum (component B), and at least one type of metal element (component C) selected from rare earth elements.

In the second aspect of the manufacturing method according to the present invention, as long as the isomerization catalyst is a metal oxide at the time of production of the olefin, the configuration before the use is not specifically limited. Organic acid salts, alkoxides, and the like may be employed insofar as they can be converted to metal oxides through a calcining treatment in an air atmosphere. For example, organic acid salt coprecipitates of Mg, Al, and Y can be converted to the above-described metal oxide by putting the coprecipitates as-is into a reactor and conducting the calcining treatment. The form of the isomerization catalyst is not specifically limited. The isomerization catalyst may be in the shape of a powder or granules, be formed into various shapes, or be fixed on a surface of a support, e.g., a honeycomb, a mesh sheet, and a filter. Furthermore, the catalyst can also be used while being supported on a surface of a catalyst carrier.

In the second aspect of the manufacturing method according to the present invention, at least a part of individual metal elements of the above-described metal oxide constitute a composite oxide. A completely single composite oxide phase may be formed or diffraction peaks attributed to the alkaline earth metal oxide, alumina, the rare-earth metal oxide, and the like may be present together in the X-ray diffraction measurement. In this regard, the metal oxide is not necessarily formed from only oxides of the metal elements, and hydroxides and carbonates may be contained.

The metal element selected from alkaline earth metal elements in the second aspect of the manufacturing method according to the present invention is the same metal element as that selected from alkaline earth metal elements in the above-described first aspect.

The metal element selected from rare earth elements in the second aspect of the manufacturing method according to the present invention is the same metal element as that selected from rare earth elements in the above-described first aspect.

In the second aspect of the manufacturing method according to the present invention, the amount of alkaline earth metal (component A) contained in the above-described isomerization catalyst is not specifically limited. However, the upper limit value is preferably 80%, more preferably 75%, and still more preferably 70% on a mole fraction basis, where a total of metal elements of the components A, B, and C is assumed to be 100%. In this regard, the lower limit value is preferably 30%, and further preferably 45%.

In the second aspect of the manufacturing method according to the present invention, the amount of aluminum (component B) contained in the above-described isomerization catalyst is not specifically limited as well. However, the upper limit value is preferably 50%, and further preferably 45% on a mole fraction basis described above. In this regard, the lower limit value is preferably 9%, more preferably 10% and still more preferably 15%.

In the second aspect of the manufacturing method according to the present invention, the amount of rare-earth metal (component C) contained in the above-described isomerization catalyst is not specifically limited as well. However, the lower limit value is preferably 5%, and further preferably 10% on a mole fraction basis described above. In this regard, the upper limit value is preferably 50%, and further preferably 20%.

Moreover, in the second aspect of the manufacturing method according to the present invention, the specific surface area of the isomerization catalyst is not specifically limited as well, but is usually 10 m²/g or more, and preferably 10 to 300 m²/g. In this regard, the lower limit value is more preferably 30 m²/g, and further preferably 50 m²/g.

In the second aspect of the manufacturing method according to the present invention, the method for manufacturing the above-described isomerization catalyst is not specifically limited, and any method of, for example, an impregnation method, an incipient wetness method, a spraying method, a coprecipitation method, a sol-gel method, and a thermal decomposition method (from chlorides, organometals, organic acid salts, and the like) may be employed, insofar as it is the method for preparing a solid catalyst. Among the above-described preparation methods, the coprecipitation method, the sol-gel method, or the thermal decomposition method (from chlorides, organometals, organic acid salts, and the like) is preferable because a desirable mixed state of the alkaline earth metal, aluminum, and the rare-earth metal can be formed easily. As for the precursor of the isomerization catalyst (hereafter referred to as a "catalyst precursor" appropriately), hydroxides, organic acid salts, carbonates, nitrates, and the like suitable for the method for manufacturing the above-described isomerization catalyst can be used.

In the case where production is conducted by the coprecipitation method, a compound, at least a part of which has a layered double hydroxide (LDH) structure, is favorably used as the catalyst precursor. The layered double hydroxides (LDH is a contraction of Layered Double Hydroxides) represent a compound group, in which constituent metal ions have a plurality of valences, hydroxide ions are contained as counter ions of the metals, and furthermore, the regularity of being layered is provided. For example, hydrotalcite is applicable. Regarding the three components according to the present invention, the alkaline earth metals are M²⁺ ions and aluminum and the rare-earth metals are M³⁺ ions. Therefore, a layered double hydroxide (LDH) structure may be formed from metal ions having two valences, that is, a valence of +2 and a valence of +3.

In the second aspect of the manufacturing method according to the present invention, the catalyst precursor, at least a part of which has a layered double hydroxide (LDH) structure, has advantages that, for example, the individual components are expected to form a mixed state at an atom level easily and the filterability of precipitates is good. The above-described catalyst precursor may be prepared by any publicly known method, in which the layered double hydroxide (LDH) can be formed. Here, it is preferable that the preparation is conducted within the range, in which 30% to 75% of component A, 10% to 50% of component B, and 5% to 50% of component C are contained on a mole fraction basis, where a total of the above-described three components is assumed to be 100%, because the layered double hydroxide (LDH) is formed easily. The range, in which 45% to 70% of component A, 15% to 45% of component B, and 10% to 20% of component C are contained, is further preferable.

In the second aspect, the above-described isomerization catalyst can be obtained from the above-described catalyst precursor by, for example, conducting calcining at a temperature of 300°C to 800°C for 0.1 to 24 hours.

In general, the proportions of the metal elements of the components A, B and C remain unchanged during the conversion from the catalyst precursor to the isomerization catalyst. Thus, it is possible to equate the proportions (mole fractions) of the metal elements of the components A, B and C in the catalyst precursor with those in the isomerization catalyst obtained from the catalyst precursor. The mole fractions of the catalyst precursor or of the isomerization catalyst are obtainable by ICP emission spectroscopic analysis.

According to the above-described second aspect of the manufacturing method of the present invention, double bond isomerization of the raw material olefins can be facilitated and side reactions of the metathesis reaction can be suppressed. As a result, a desired product can be obtained from the raw material olefins at a high selectivity.

The present inventors have invented the method for manufacturing an olefin according to the above-described first aspect and the second aspect, wherein a desired product can be obtained at selectivity higher than that of the conventional method. Furthermore, the present inventors have invented the method for manufacturing an olefin according to the third aspect, described below, wherein deterioration of the isomerization activity is suppressed and a desired product can be obtained at a high selectivity for a long term.

That is, the third aspect of the manufacturing method according to the present invention is a method for manufacturing an olefin, the method including two reaction treatments of the metathesis reaction and the double bond isomerization reaction and being **characterized in that**
the metathesis catalyst is a catalyst (a) containing at least one type of metal element selected from the group consisting of tungsten, molybdenum, and rhenium,
the isomerization catalyst is a metal oxide catalyst (b) containing at least one type of metal element selected from alkaline earth metal elements and rare earth elements, and
a catalyst (c) containing alumina together with the above-described metathesis catalyst and/or the above-described isomerization catalyst.

The metathesis catalyst (catalyst (a)) in the third aspect of the manufacturing method according to the present invention is the same metathesis catalyst as that in the above-described first aspect.

In the third aspect of the manufacturing method according to the present invention, the isomerization catalyst (catalyst (b)) is not specifically limited insofar as the catalyst contains at least one type of metal element selected from alkaline earth metal elements and rare earth elements. It is preferable that the above-described isomerization catalyst (catalyst (b)) is a metal oxide catalyst containing at least one type of metal element selected from alkaline earth metal elements and rare earth elements because of the stability under the olefin production condition and the catalyst regeneration treatment condition.

In the third aspect of the manufacturing method according to the present invention, the metal oxide is not necessarily formed from only an oxide of the metal element, and a hydroxide and a carbonate may be contained. Furthermore, as long as a metal oxide is ready to use at the time of production of the olefin, the configuration before the use is not specifically limited. Organic acid salts, alkoxides, and the like may be employed insofar as they can be converted to metal oxides through a calcining treatment in an air atmosphere. For example, organic acid salt coprecipitates of Mg and Y can be converted to the above-described metal oxides by putting the coprecipitates as-is into a reactor and conducting the calcining treatment.

The metal element selected from alkaline earth metal elements in the third aspect of the manufacturing method according to the present invention is the same metal element as that selected from alkaline earth metal elements in the above-described first aspect.

The metal element selected from rare earth elements in the third aspect of the manufacturing method according to the present invention is the same metal element as that selected from rare earth elements in the above-described first aspect.

In the third aspect of the manufacturing method according to the present invention, examples of isomerization catalysts (catalysts (b)) include magnesium oxide, calcined products of hydrotalcite containing magnesium and aluminum, yttrium oxide, metal oxides containing magnesium and yttrium, and composite oxides containing three components of magnesium, aluminum, and yttrium, although not limited to them.

In the third aspect of the manufacturing method according to the present invention, the catalyst (c) used together with the above-described metathesis catalyst (catalyst (a)) and/or the above-described isomerization catalyst (catalyst (b)) is not specifically limited insofar as alumina is contained as a component. The above-described catalyst (c) is preferably a catalyst having a specific surface area of 50 m²/g or more, and further preferably a catalyst having a specific surface area of 100 m²/g or more. The upper limit of the specific surface area is not specifically limited, but usually 350 m²/g or less. Moreover, the catalyst (c) may contain metal elements other than aluminum, e.g., alkali metals, alkaline earth metals, rare earth elements, and the like.

The methods for manufacturing the above-described catalyst (a), the above-described catalyst (b), and the above-described catalyst (c) are not specifically limited, and any method of, for example, an impregnation method, an incipient wetness method, a spraying method, a precipitation method, a coprecipitation method, a sol-gel method, and a thermal decomposition method (from chlorides, organometals, organic acid salts, and the like) may be employed, insofar as it is the method for preparing a solid catalyst. The form of the catalyst is not specifically limited. The catalyst may be in the shape of a powder or granules, be formed into various shapes, or be fixed on a surface of a support, e.g., a honeycomb, a mesh sheet, and a filter. Furthermore, the catalyst can also be used while being supported on a surface of a catalyst carrier.

Moreover, the above-described catalyst (a), the above-described catalyst (b), and the above-described catalyst (c) may be independently made into the form of the above-described catalyst, or the state of mixture of at least two catalysts may be made into the form of the above-described catalyst. The above-described catalyst (a), the above-described catalyst (b), and the above-described catalyst (c) may be formed bodies made independently, and the above-described individual formed bodies may be present together in one reactor. Alternatively, the above-described catalyst (a) may be a formed body (i) made independently, the above-described catalyst (b) and the above-described catalyst (c) may constitute a formed body (ii) by being mixed with each other, and the above-described formed body (i) and the above-described formed body (ii) may be present together in one reactor.

In the case where the individual catalysts are used as formed bodies, the forming conditions suitable for the individual catalysts can be adopted independently. Consequently, in many cases, the individual catalysts are made into the formed bodies independently. When the individual catalysts are independently made into the formed bodies, it is possible to sort out and collect the used catalysts and thus it is easy to reuse the individual metals such as rare earth metals.

According to the above-described third aspect of the manufacturing method of the present invention, the activity of the metathesis catalyst can be maintained for a long term and, in addition, deterioration of performance of the isomerization catalyst can be suppressed, so that a desired product can be obtained at a high selectivity for a long term.

It is preferable that raw material olefins including at least an olefin having the carbon number of 3 or more is used in the manufacturing method according to the present invention. All components of the above-described raw material olefins are not necessarily an olefin having the carbon number of 3 or more, and ethylene, paraffin, and the like may be contained.

The olefin having the carbon number of 3 or more is any one of straight chain olefin, branched olefin, and cyclic olefin, and is not specifically limited except that a substituent acting as a catalyst poison to the metathesis catalyst and the isomerization catalyst is not contained. Examples of substituents acting as catalyst poisons include functional groups containing a hetero atom in the structure, e.g., a hydroxyl group, a carbonyl group, an alkoxy group, an amino group, a nitro group, and a thiol group, and substituents containing a hetero ring as a partial structure, e.g., furan, pyridine, and thiophene. The olefin having the carbon number of 3 or more is not specifically limited. Examples thereof can include propylene, 1-butene, 2-butene, isobutene, 1-pentenes, 2-pentene, 2-methyl-1-butene, 2-methyl-2-butene, 3-methyl-1-butene, 1-hexene, 2-hexene, 3-hexene, 2-methyl-1-pentene, 2-methyl-2-pentene, 4-methyl-1-pentene, 4-methyl-2-pentene, 3-methyl-1-pentene, 3-methyl-2-pentene, cyclopentene, and cyclohexene.

The metathesis reaction is a reaction, in which from two molecules of the same type or different types of olefins, two molecules of olefins having a structure different from them are generated. That is, in the case where from different types of olefins, production of an olefin different from them is conducted, basically, it is enough that an equal mole of each olefin is present in the raw material olefins. However, it is possible that one type of olefins is allowed to be present excessively so as to suppress the reaction between the same type of olefins. In particular, regarding the metathesis reaction of ethylene with each other, an olefin different from ethylene is not generated. Therefore, in the case where one of the different types of olefins is ethylene, in general, ethylene is used excessively. For example, in the case where propylene is produced from ethylene and n-butene serving as raw material olefins, the molar ratio of ethylene/n-butene is preferably 1 to 50, and more preferably 1 to 5. The manufacturing method according to the present invention can be executed out of the above-described range. However, the above-described range is favorable from the viewpoint of both the commercial demand for reduction in usage of ethylene, which is an expensive feedstock relative to butene, and the above-described suppressing effect.

The metathesis catalyst and the isomerization catalyst are sensitive to catalyst poisons, and in general, removal of catalyst poisons from the above-described raw material olefins is conducted by disposing a guard bed upstream to a reactor. Examples of catalyst poisons include water, carbon dioxide, oxygen-containing compounds (for example, ketones, ethers, and alcohols), sulfur-containing compounds, and nitrogen-containing compounds. There is no limitation as to whether the guard bed is located immediately in front of the reactor or is located considerably upstream to the reactor as long as catalyst poisons are removed and, thereafter, a fresh catalyst poison is not mixed.

It is preferable that the raw material olefins including an olefin having the carbon number of 3 or more contain n-butene. Furthermore, it is preferable that the raw material olefins further contain ethylene.

In the case where the raw material olefins containing n-butene as the olefin having the carbon number of 3 or more are used, examples of industrially usable raw materials include a C₄ fraction and a C₄ raffinate obtained through steam cracking of naphtha, butane (converted to butene through dehydrogenation), ethylene (converted to butene through dimerization reaction in a liquid phase or a gas phase), propylene (converted to ethylene and butene through metathesis), and butadiene (converted to butene through selective hydrogenation).

In general, the metathesis catalyst and the isomerization catalyst are used for reactions after being activated through a pretreatment. In the manufacturing method according to the present invention as well, a specific condition is not necessary, and a publicly known treatment condition may be adopted. Moreover, regarding the catalyst (c) in the third aspect of the manufacturing method according to the present invention as well, a specific condition is not necessary, and a publicly known treatment condition may be adopted.

In general, the metathesis catalyst is used after being subjected to a calcining treatment in the presence of oxygen, a calcining treatment in an inert gas atmosphere, or a reduction treatment. The calcining treatment in the presence of oxygen is conducted usually at 350°C to 800°C in the case where conversion of a tungsten, molybdenum, or rhenium species contained in the metathesis catalyst to an oxide is necessary. The calcining treatment in an inert gas atmosphere is conducted usually at 350°C to 800°C. In addition, the reduction treatment is conducted at 300°C to 750°C while carbon monoxide or hydrogen is supplied as a reducing agent.

On the other hand, in general, the isomerization catalyst is calcined at 300°C to 800°C in an inert gas stream, and dehydration, decarboxylation, or removal of adhered materials is conducted. Calcining may be conducted at 350°C to 800°C in the presence of oxygen in order to convert to the above-described metal oxide. Furthermore, in the case where the metathesis catalyst and the isomerization catalyst are used by being filled into the same reactor, the isomerization catalyst may be treated under the above-described reduction treatment condition. Moreover, regarding the pretreatment of the catalyst (c) in the third aspect of the manufacturing method according to the present invention, the catalyst (c) may be present together in the pretreatment of the metathesis catalyst and/or the isomerization catalyst so as to be subjected to the same treatment. A specific treatment for the catalyst (c) may not be conducted.

In addition, the catalyst exhibiting deteriorated performance due to the use for production of an olefin is subjected to the calcining treatment in the presence of oxygen or the calcining treatment in an inert gas atmosphere so as to remove catalyst poisons adhered to the catalyst surface, heavy materials formed on the catalyst surface under the reaction condition, and the like and, therefore, can be used repeatedly. This treatment (hereafter referred to as a "regeneration treatment") is conducted usually at a temperature of 300°C to 800°C regardless of the atmosphere. The regeneration treatment temperature may be the same as or different from that in the above-described pretreatment condition.

The metathesis reaction condition is not specifically limited insofar as the reaction proceeds under that condition. The temperature is 0°C to 600°C, and preferably 20°C to 500°C. In the case where the metal element contained in the metathesis catalyst is tungsten, the reaction is effected frequently in a medium to high temperature range of the above-described temperature range. As for molybdenum, the reaction is effected frequently in a medium temperature range of the above-described temperature range, and as for rhenium, the reaction is effected frequently in a low to medium temperature range. In the case where hydrogen is also supplied together with the raw material olefins, the reaction may be effected in a still lower temperature range. The pressure is 0 (atmospheric pressure) to 20 MPa, and preferably 0 to 10 MPa on a gauge pressure basis. The feed rate of the raw material olefins is preferably 1 to 500 hr⁻¹, and further preferably 1 to 250 hr⁻¹ on a WHSV basis with reference to the weight of the metathesis catalyst. In the case where hydrogen is supplied together with the raw material olefins, the amount of supply of hydrogen is 0.05 to 10 percent by volume, and preferably 0.08 to 5 percent by volume relative to the total amount of supply of the raw material olefins and hydrogen.

The isomerization reaction condition is not specifically limited insofar as the reaction proceeds under that condition. The temperature is 0°C to 600°C, and preferably 20°C to 500°C. The pressure is 0 (atmospheric pressure) to 20 MPa, and preferably 0 to 10 MPa on a gauge pressure basis. The feed rate of the raw material olefins is 0.1 to 1,000 hr⁻¹, and preferably 0.1 to 500 hr⁻¹ on a WHSV basis with reference to the weight of the isomerization catalyst.

The manufacturing method according to the present invention is preferably a method for manufacturing an olefin including two steps of obtaining an olefin from the same type or different types of olefins among raw material olefins including an olefin having the carbon number of 3 or more, the resulting olefin being different from the same type or the different types of olefins, in a reactor containing a metathesis catalyst, as a metathesis reaction step and isomerizing an olefin to shift the position of a double bond within the molecule in a reactor which contains an isomerization catalyst and in which raw material olefins are present which are the same as or different from the raw material olefins and include an olefin having the carbon number of 3 or more. In addition to these steps, necessary steps, e.g., a catalyst poison reduction step and a separation and refining step, may be added depending on raw material olefins to be used and reaction products.

In the manufacturing method according to the present invention, the metathesis reaction step and the isomerization reaction step may be conducted with separate reactors or be conducted with one reactor. An advantage of usage of separate reactors is believed to be that a reaction condition most suitable for each of the two reactions can be set. On the other hand, an advantage of usage of one reactor is believed to be that equipment can be simplified and this is highly economical. The reaction system is not specifically limited and any reaction system using a heterogeneous catalyst, e.g., a fixed bed a fluidized bed, and a suspension bed, can be selected. In the case where ethylene, propylene, butenes, pentenes, and the like are used as the raw material olefins, a gas phase reaction is preferable and, in many cases, the fixed bed and the fluidized bed are selected.

In the manufacturing method according to the present invention, it is preferable that hydrogen is also supplied together with the raw material olefins, and it is preferable that hydrogen is also supplied together with the raw material in at least one of the above-described isomerization reaction step and the above-described metathesis reaction.

In the case where the metathesis reaction and the isomerization reaction are effected with separate reactors, production may be conducted while an isomerization reactor and a metathesis reactor are arranged in that order from the upstream or, conversely, the metathesis reactor and the isomerization reactor may be arranged in that order from the upstream. In the present invention, the isomerization reaction step and the metathesis reaction step can be conducted with separate reactors, and the olefin generated in the isomerization reaction step can be used as at least a part of the raw material olefins for the metathesis reaction step. For example, in the case where propylene is produced from ethylene and n-butene, a process can be constructed, in which raw material olefins containing n-butene are supplied to an isomerization reactor so as to be converted to raw material olefins rich in 2-butene, ethylene is mixed therein, the resulting raw material olefins are supplied to a metathesis reactor so as to synthesize propylene from ethylene and 2-butene, then ethylene, propylene, and butene are separated by a method of distillation or the like and, thereafter, unreacted ethylene and n-butene are circulated and supplied to the isomerization reactor or the metathesis reactor.

Furthermore, in the third aspect of the manufacturing method according to the present invention, for example, in the case where propylene is produced from ethylene and n-butene, a process can be constructed, in which raw material olefins are supplied to a reactor filled with physically mixed formed body of the catalyst (a), formed body of the catalyst (b), and formed body of the catalyst (c), so as to effect isomerization of 1-butene to 2-butene and effect propylene synthesis through the metathesis reaction between 2-butene and ethylene, then ethylene, propylene, and butene are separated by a method of distillation or the like and, thereafter, unreacted ethylene and n-butene are circulated and supplied to the reactor.

In the first and the second aspects of the manufacturing method according to the present invention, in the case where the metathesis reaction and the isomerization reaction are effected with one reactor, as for a fixed bed reactor, it is favorable that the metathesis catalyst and the isomerization catalyst are physically mixed and disposed in the inside of the reactor or disposed while being laminated in the order of the isomerization catalyst and the metathesis catalyst from the upstream. As for a fluidized bed reactor, it is favorable that the physically mixed catalyst is used. The weight ratio of the isomerization catalyst to the metathesis catalyst in the usage is not specifically limited. However, the ratio of isomerization catalyst weight/metathesis catalyst weight is usually 0.1 or more, preferably 0.3 to 20, and further preferably 0.5 to 10.

Moreover, in the third aspect of the manufacturing method according to the present invention, in the case where the catalyst (a), the catalyst (b), and the catalyst (c) are present together in the inside of the same reactor, the catalysts in the forms suitable for the adopted reaction system may be present while being physically mixed or the individual catalysts may be laminated separately. Regarding the fixed bed reactor, in the case where the catalyst (a), the catalyst (b), and the catalyst (c) are formed bodies produced independently of each other, examples of methods include methods, in which (i) the individual formed bodies are physically mixed and allowed to be present together in the inside of the reactor, (ii) the individual formed bodies are laminated in the order of the catalyst (c), the catalyst (b), and the catalyst (a) from the upstream, and (iii) the individual formed bodies are laminated in the order of a physically mixed layer of formed bodies of the catalyst (b) and the catalyst (c) and a single layer of the catalyst (a) from the upstream. In addition, regarding the suspension bed reactor, examples thereof include a method in which the catalyst (a), the catalyst (b), and the catalyst (c) are used by being independently made granular and being suspended.

In the third aspect of the manufacturing method according to the present invention, the weight ratio of the individual catalysts in the inside of the reactor is not specifically limited. However, the ratio of the total weight of catalyst (b) and catalyst (c)/ the weight of catalyst (a) is usually 0.1 or more, preferably 0.3 to 20, and further preferably 0.5 to 10. The ratio of the weight of catalyst (b)/catalyst (c) is usually 0.01 to 100, preferably 0.1 to 10, and further preferably 1 to 7.

In a further specific aspect according to the present invention, the above-described isomerization catalyst and the metathesis catalyst are supplied to a reactor and in the reactor, raw material olefins (olefin mixture) containing ethylene and 1-butene are supplied so as to convert 1-butene, to 2-butene by the isomerization catalyst and, subsequently, propylene is produced from 2-butene resulting from the conversion and ethylene by the metathesis catalyst. In addition, in order to maintain the activity of the metathesis catalyst at a high level for a long term, a catalyst containing alumina may be further supplied to the reactor.

### [EXAMPLES]

The present invention will be further specifically described below with reference to examples, although the present invention is not limited to them.

### [Preparation of catalyst]

### (Preparation example 1-1) Preparation of Metathesis catalyst WQ-15

Ammonium metatungstate was supported on a SiO₂ carrier (Fuji Silysia Chemical Ltd., CARiACT Q-15) through impregnation and, thereafter, calcining was conducted in an air atmosphere at 500°C so as to obtain WO₃/SiO₂ catalyst. This was pulverized and classified into the size of 150 to 500 µm so as to serve as WQ-15.

### (Preparation example 1-2) Preparation 1 of Isomerization catalyst containing Mg and Y

As for an isomerization catalyst containing Mg and Y, the ratio of amount of metal nitrate to be used was changed and, thereby, a catalyst having a Mg/Y molar ratio = 0.1, 0.5, 1, 3, 5, or 7 was prepared.

A mixed aqueous solution of magnesium nitrate and yttrium nitrate and 25% ammonia water were prepared and transferred to a flask containing water therein, followed by mixing. Here, the feed rate of ammonia water was controlled in such a way as to keep pH of the liquid in the flask at 10. After mixing, ammonia water was added appropriately until a change in pH of the liquid in the flask stopped, followed by aging. The resulting precipitates were washed by repeating filtration and redispersion into water. The resulting wet cake was dried and, thereafter, was calcined in an air atmosphere at 550°C so as to obtain a metal oxide containing Mg and Y. This was made into a powder and, thereafter, compression molding was conducted. Pulverization and classification into the size of 150 to 500 µm were conducted so as to obtain an isomerization catalyst containing Mg and Y. The Mg/Y molar ratio was calculated on the basis of analysis of the isomerization catalyst with ICP emission spectroscopic analysis device (VISTA-PRO, manufactured by Seiko Instruments Inc.).

The catalysts having Mg/Y molar ratios of 0.1, 0.5, 1, 3, 5, and 7 were assumed to be MY-01, MY-05, MY-10, MY-30, MY-50, and MY-70, respectively.

### (Preparation example 1-3) Preparation 2 of Isomerization catalyst containing Mg and Y

A mixed aqueous solution of magnesium nitrate and yttrium nitrate and sodium hydroxide aqueous solution were prepared. Subsequently, a solution, in which sodium carbonate was dissolved in such an amount that allows carbonate ion to become equal moles relative to yttrium salts, was prepared in a flask, and the nitrate aqueous solution and the sodium hydroxide aqueous solution were transferred thereto, followed by mixing, while the temperature was kept at 60°C. Here, the feed rate of sodium hydroxide aqueous solution was controlled in such a way as to keep pH of the liquid in the flask at 10. After mixing was completed, heating was further conducted so as to conduct aging with reflux for 2 hours. The resulting precipitates were washed by repeating filtration and redispersion into water. The resulting wet cake was dried and, thereafter, was calcined in an air atmosphere at 550°C so as to obtain a metal oxide containing Mg and Y. This was made into a powder and, thereafter, compression molding was conducted. Pulverization and classification into the size of 150 to 500 µm were conducted so as to obtain MY-3N. The Mg/Y molar ratio was calculated on the basis of analysis with ICP and the result was 3.

### (Preparation example 1-4) Preparation of Isomerization catalyst containing Mg and La

A mixed aqueous solution of magnesium nitrate and lanthanum nitrate and a mixed aqueous solution of potassium hydroxide and potassium carbonate were prepared and transferred to a flask containing water therein, followed by mixing. Here, the feed rate of potassium salt aqueous solution was controlled in such a way as to keep pH of the liquid in the flask at 10. After mixing, the potassium salt aqueous solution was added appropriately until a change in pH of the liquid in the flask stopped, followed by aging. The resulting precipitates were washed by repeating filtration and redispersion into water. The resulting wet cake was dried and, thereafter, was calcined in an air atmosphere at 550°C so as to obtain a metal oxide containing Mg and La. This was made into a powder and, thereafter, compression molding was conducted. Pulverization and classification into the size of 150 to 500 µm were conducted so as to obtain Isomerization catalyst ML-30. The Mg/La molar ratio was 3 on the basis of analysis with ICP.

### (Preparation example 1-5) Preparation of Isomerization catalyst REF-1

A powder of magnesium oxide (Kyowa Chemical Industry Co., Ltd., KM-150) was compression-molded. Thereafter, pulverization and classification into the size of 150 to 500 µm were conducted, and calcining was conducted in an air atmosphere at 550°C, so as to obtain REF-1.

### (Examples 1-1 to 1-8, Comparative example 1-1)

### [Production 1-1 of propylene from ethylene and 1-butene] (Reactor)

As for a reactor, the center of a stainless steel single pipe reactor was filled with 50 mg of the metathesis catalyst WQ-15 and 150 mg of the isomerization catalyst, and alumina balls serving as preheat retaining layers were filled on and under the catalysts.

### (Production of propylene)

The metathesis catalyst and the isomerization catalyst in the state of being filled in the reactor were subjected to a calcining treatment and a reduction treatment in advance and, furthermore, purging with nitrogen was conducted. Thereafter, the temperature of the catalyst bed was lowered to a reaction temperature. Subsequently, a mixed gas of 1-butene refined through an activated alumina column and high-purity ethylene was supplied as raw material olefins so as to effect a flow reaction at atmospheric pressure. Here, the reaction temperature was specified to be 175°C, and the total feed rate of olefins was specified to be WHSV = 40 hr⁻¹ relative to the weight of WQ-15. Furthermore, hydrogen in an amount of 10 percent by volume of the total amount of supply of olefins and hydrogen was mixed with the raw material olefins and supplied to the reactor. Regarding a gas at a reactor outlet, the concentrations of ethylene, propylene, butenes, and pentenes were analyzed by using FID-GC so as to determine the butene conversion, the propylene selectivity, and the 2-butene/1-butene molar ratio (expressed as 2B/1B in Table) of the outlet gas.

Table 1-1 shows the results of the flow reaction by using the isomerization catalyst prepared in Preparation examples 1-2 to 1-5 at the points in time after 8 hours and 15 hours from the start of the reaction. As is indicated by these results, regarding the manufacturing method according to the present invention, the selectivity is improved because of the highly active isomerization catalyst as compared with that in the manufacturing method by using the publicly known catalyst.

**[Table 1-1]**

| | | | | | | | | |
|---|---|---|---|---|---|---|---|---|
| Production of propylene at atmospheric pressure, 175°C, and addition of hydrogen of 10% | | | | | | | | |

| | Metathesis catalyst | Isomerization catalyst | Butene conversion [%] | | Propylene selectivity [%] | | 2B/1B | |
|---|---|---|---|---|---|---|---|---|
| | | | 8h | 15h | 8h | 15h | 8h | 15h |
| Example 1-1 | WQ-15 | MY-01 | 65.8 | 65.4 | 92.7 | 92.1 | 5.9 | 5.8 |
| Example 1-2 | WQ-15 | MY-05 | 66.0 | 64.9 | 93.2 | 92.3 | 6.2 | 6.0 |
| Example 1-3 | WQ-15 | MY-10 | 65.5 | 63.0 | 93.9 | 92.7 | 6.4 | 6.3 |
| Example 1-4 | WQ-15 | MY-30 | 65.4 | 66.0 | 93.9 | 94.2 | 6.5 | 6.5 |
| Example 1-5 | WQ-15 | MY-50 | 63.2 | 64.3 | 92.8 | 93.3 | 6.5 | 6.5 |
| Example 1-6 | WQ-15 | MY-70 | 66.2 | 65.4 | 94.2 | 93.8 | 6.6 | 6.5 |
| Example 1-7 | WQ-15 | MY-3N | 66.2 | 66.8 | 94.3 | 94.7 | 6.6 | 6.6 |
| Example 1-8 | WQ-15 | ML-30 | 64.9 | 65.1 | 92.9 | 92.6 | 6.3 | 6.1 |
| Comparative example 1-1 | WQ-15 | REF-1 | 34.5 | 8.9 | 54.6 | 44.9 | 0.6 | 0.5 |

### (Examples 1-9 to 1-10, Comparative example 1-2)

### [Production 1-2 of propylene from ethylene and 1-butene] (Reactor)

As for a reactor, the center of a stainless steel single pipe reactor was filled with 0.2 g of the metathesis catalyst WQ-15 and 0.2 g of the isomerization catalyst, and alumina balls serving as preheat retaining layers were filled on and under the catalysts.

### (Production of propylene)

The metathesis catalyst and the isomerization catalyst in the state of being filled in the reactor were subjected to a calcining treatment and a reduction treatment in advance and, furthermore, purging with nitrogen was conducted. Thereafter, the temperature of the catalyst bed was lowered to a reaction temperature. Subsequently, a mixed gas of 1-butene refined through an activated alumina column and high-purity ethylene was supplied as raw material olefins so as to effect a flow reaction at atmospheric pressure. Here, the reaction temperature was specified to be 300°C, and the total feed rate of olefins was specified to be WHSV = 10 hr⁻¹ relative to the weight of WQ-15. Regarding a gas at a reactor outlet, the concentrations of ethylene, propylene, butenes, and pentenes were analyzed by using FID-GC so as to determine the butene conversion, the propylene selectivity, and the 2-butene/1-butene molar ratio (expressed as 28/1B in Table) of the outlet gas.

Table 1-2 shows the results of the flow reaction by using MY-70, ML-30, or REF-1 as the isomerization catalyst at the points in time after 8 hours and 15 hours from the start of the reaction. As is indicated by these results, regarding the manufacturing method according to the present invention, the selectivity is improved because of the highly active isomerization catalyst as compared with that in the manufacturing method by using the publicly known catalyst.

**[Table 1-2]**

| | | | | | | | | |
|---|---|---|---|---|---|---|---|---|
| Production of propylene at atmospheric pressure and 300°C without hydrogen | | | | | | | | |

| | Metathesis catalyst | Isomerization catalyst | Butene conversion [%] | | Propylene selectivity [%] | | 2B/1B | |
|---|---|---|---|---|---|---|---|---|
| | | | 8h | 15h | 8h | 15h | 8h | 15h |
| Example 1-9 | WQ-15 | MY-70 | 52.7 | 49.3 | 84.6 | 81.3 | 4.0 | 3.5 |
| Example 1-10 | WQ-15 | ML-30 | 40.1 | 34.3 | 80.4 | 77.0 | 3.6 | 3.2 |
| Comparative example 1-2 | WQ-15 | REF-1 | 34.2 | 28.5 | 66.6 | 63.5 | 0.8 | 0.7 |

### [Preparation of catalyst]

### (Preparation example 2-1) Preparation of Metathesis catalyst WQ-15

As in Preparation example 1-1 described above, NQ-15 was prepared.

### (Preparation example 2-2) Preparation of Isomerization catalyst containing Mg, Al, and Y

Isomerization catalysts containing Mg, Al, and Y were prepared as described below by changing the amount of the individual metal nitrate to be used, more specifically, by using charged compositions shown in Table 2-1 on a mole fraction basis, where a total of Mg, Al, and Y is assumed to be 100%.

Preparation of 0.2 liter of mixed aqueous solution of magnesium nitrate, aluminum nitrate, and yttrium nitrate (hereafter referred to as a "metal salt solution" appropriately) was conducted in such a way that the metal ion concentration became 0.8 mol/L on a total of Mg²⁺, Al³⁺, and Y³⁺ basis. In addition, a sufficient amount of sodium hydroxide aqueous solution (hereafter referred to as a "precipitant" appropriately) having a concentration of 4 mol/L was prepared.

Then, sodium carbonate, in which the amount of carbonic acid groups was equal to the total amount of Al³⁺ and Y³⁺ in the above-described metal salt solution on a mole basis was added to a flask having 0.6 liter of water therein and was dissolved through stirring. The flask was heated while stirring was continued so as to keep the internal temperature at 60°C, and the above-described metal salt solution was dropped thereto at a constant speed over 20 minutes. Here, dropping of the above-described precipitant was started at the point in time when the pH of the liquid in the flask reached 10, so as to control the pH of the liquid in the flask at 10. The dropping of the precipitant was terminated at the point in time when the dropping of the metal salt solution was completed and, subsequently, the flask was heated and reflux was conducted for 2 hours. Thereafter, cooling was conducted to room temperature, white slurry in the inside of the flask, was filtrated, and the resulting precipitates were washed by repeating filtration and redispersion into water. Then, the resulting wet cake was dried at 130°C so as to obtain Catalyst precursors 1 to 11. Fig. 2-4 shows the X-ray diffraction patterns of the resulting Catalyst precursors 1 to 11 and, in addition, Fig. 2-5 shows a diagram for comparing the X-ray diffraction pattern of Catalyst precursor 8 with JCPDS Card No. 35-1275 Mg₂Al(OH)₇. The catalyst precursor was calcined in an air atmosphere at 550°C for 5 hours and was made into a powder. After compression molding was conducted, pulverization and classification into the size of 150 to 500 µm were conducted so as to obtain Isomerization catalysts 1 to 11 containing Mg, Al, and Y.

Table 2-1 collectively shows the BET specific surface areas of the resulting Isomerization catalysts 1 to 11 measured by a nitrogen adsorption method through the use of specific surface area/pore size distribution measurement instrument BELSORP-max-2 (BEL Japan, Inc.) and the compositional ratios obtained from the measurement results using ICP emission spectroscopic analysis device (VISTA-PRO, manufactured by Seiko Instruments Inc.).

### (Preparation example 2-3) Preparation of Isomerization catalyst 12

A powder of magnesium oxide (Kyowa Chemical Industry Co., Ltd., KM-150) was compression-molded. Thereafter, pulverization and classification into the size of 150 to 500 µm were conducted, and calcining was conducted in an air atmosphere at 550°C, so as to obtain Isomerization catalyst 12. The BET specific surface area of the resulting Isomerization catalyst 12 measured as in Preparation example 2-2 is shown in Table 2-1.

**[Table 2-1]**

| | | | | | | | | |
|---|---|---|---|---|---|---|---|---|
| Composition and specific surface area of catalyst | | | | | | | | |

| Catalyst precursor | Isomerization catalyst | Mole fraction (%, charged) | | | Mole fraction (%, analyzed value) | | | Specific surface area (m²/g) |
|---|---|---|---|---|---|---|---|---|
| | | Mg | Al | Y | Mg | Al | Y | |
| 1 | 1 | 75 | 17 | 8 | 78.47 | 14.14 | 7.39 | 108 |
| 2 | 2 | 67 | 11 | 22 | 71.13 | 9.42 | 19.95 | 86 |
| 3 | 3 | 67 | 22 | 11 | 71.26 | 18.88 | 9.86 | 130 |
| 4 | 4 | 60 | 20 | 20 | 65.49 | 16.60 | 17.91 | 135 |
| 5 | 5 | 60 | 30 | 10 | 65.47 | 25.68 | 8.85 | 133 |
| 6 | 6 | 50 | 25 | 25 | 54.62 | 21.74 | 23.64 | 128 |
| 7 | 7 | 50 | 33 | 17 | 55.82 | 28.92 | 15.26 | 122 |
| 8 | 8 | 50 | 40 | 10 | 58.27 | 31.85 | 9.88 | 139 |
| 9 | 9 | 40 | 40 | 20 | 47.94 | 31.80 | 20.26 | 160 |
| 10 | 10 | 37 | 50 | 13 | 50.98 | 35.08 | 13.94 | 150 |
| 11 | 11 | 30 | 50 | 20 | 42.32 | 33.32 | 24.36 | 143 |
| - | 12 | 100 | 0 | 0 | - | - | - | 150 |

### (Examples 2-1 to 2-11, Comparative example 2-1)

### [Production 2-1 of propylene from ethylene and 1-butene] (Reactor)

As for a reactor, the center of a stainless steel single pipe reactor was filled with 50 mg of the metathesis catalyst WQ-15 and 150 mg of the isomerization catalyst, and alumina balls serving as preheat retaining layers were filled on and under the catalysts.

### (Production of propylene)

The metathesis catalyst and the isomerization catalyst in the state of being filled in the reactor were subjected to a calcining treatment and a reduction treatment in advance and, furthermore, purging with nitrogen was conducted. Thereafter, the temperature of the catalyst bed was lowered to a reaction temperature. Subsequently, a mixed gas of 1-butene refined through an activated alumina column and high-purity ethylene was supplied as raw materials olefins so as to effect a flow reaction at atmospheric pressure. Here, the reaction temperature was specified to be 175°C, and the total feed rate of olefins was specified to be WHSV = 40 hr⁻¹ relative to the weight of WQ-15. Furthermore, hydrogen in an amount of 10 percent by volume of the total amount of supply of olefins and hydrogen was mixed with the raw material olefins and supplied to the reactor. Regarding a gas at a reactor outlet, the concentrations of ethylene, propylene, butenes, and pentenes were analyzed by using FID-GC so as to determine the butene conversion, the propylene selectivity, and the 2-butene/1-butene molar ratio (expressed as 2B/1B in Table) of the outlet gas.

Table 2-2 and Figs. 2-1 to 2-3 show the results of the flow reaction by using Isomerization catalysts 1 to 12 at the points in time after 8 hours and 15 hours from the start of the reaction. As is indicated by these results, regarding the manufacturing method according to the present invention, the selectivity is improved because of the highly active isomerization catalyst as compared with that in the manufacturing method by using the publicly known catalyst.

**[Table 2-2]**

| | | | | | | | | |
|---|---|---|---|---|---|---|---|---|
| Production of propylene at atmospheric pressure, 175°C, and addition of hydrogen of 10% | | | | | | | | |

| | Metathesis catalyst | Isomerization catalyst | Butene conversion [%] | | Propylene selectivity [%] | | 2B/1B | |
|---|---|---|---|---|---|---|---|---|
| | | | 8h | 15h | 8h | 15h | 8h | 15h |
| Example 2-1 | WQ-15 | 1 | 53.0 | 31.7 | 85.4 | 73.4 | 5.2 | 3.1 |
| Example 2-2 | WQ-15 | 2 | 65.3 | 64.7 | 93.8 | 93.1 | 6.4 | 6.4 |
| Example 2-3 | WQ-15 | 3 | 60.2 | 57.8 | 90.3 | 88.1 | 6.0 | 5.2 |
| Example 2-4 | WQ-15 | 4 | 60.3 | 59.6 | 90.7 | 90.1 | 6.4 | 6.3 |
| Example 2-5 | WQ-15 | 5 | 59.7 | 58.2 | 89.1 | 87.3 | 5.6 | 5.1 |
| Example 2-6 | WQ-15 | 6 | 65.2 | 63.8 | 93.0 | 91.6 | 6.4 | 6.2 |
| Example 2-7 | WQ-15 | 7 | 65.8 | 66.5 | 93.3 | 93.4 | 6.1 | 6.1 |
| Example 2-8 | WQ-15 | 8 | 62.4 | 56.5 | 90.1 | 84.7 | 5.8 | 4.9 |
| Example 2-9 | WQ-15 | 9 | 58.1 | 45.9 | 87.6 | 79.6 | 5.6 | 4.5 |
| Example 2-10 | WQ-15 | 10 | 64.9 | 64.4 | 92.2 | 90.6 | 6.0 | 5.5 |
| Example 2-11 | WQ-15 | 11 | 62.6 | 62.3 | 90.8 | 89.4 | 5.5 | 5.0 |
| Comparative example 2-1 | WQ-15 | 12 | 34.5 | 8.9 | 54.6 | 44.9 | 0.6 | 0.5 |

### (Examples 2-12 to 2-14, Comparative example 2-2)

### [Production 2-2 of propylene from ethylene and 1-butene] (Reactor)

As for a reactor, the center of a stainless steel single pipe reactor was filled with 0.2 g of the metathesis catalyst WQ-15 and 0.2 g of the isomerization catalyst, and alumina balls serving as preheat retaining layers were filled on and under the catalysts.

### (Production of propylene)

The metathesis catalyst and the isomerization catalyst in the state of being filled in the reactor were subjected to a calcining treatment and a reduction treatment in advance and, furthermore, purging with nitrogen was conducted. Thereafter, the temperature of the catalyst bed was lowered to a reaction temperature. Subsequently, a mixed gas of 1-butene refined through an activated alumina column and high-purity ethylene was supplied as raw material olefins so as to effect a flow reaction at atmospheric pressure. Here, the reaction temperature was specified to be 300°C, and the total feed rate of olefins was specified to be WHSV = 10 hr⁻¹ relative to the weight of WQ-15. Regarding a gas at a reactor outlet, the concentrations of ethylene, propylene, butenes, and pentenes were analyzed by using FID-GC so as to determine the butene conversion, the propylene selectivity, and the 2-butene/1-butene molar ratio (expressed as 2B/1B in Table) of the outlet gas.

Table 2-3 shows the results of the flow reaction by using Isomerization catalysts 1, 5, 7, or 12 at the points in time after 8 hours and 15 hours from the start of the reaction. As is indicated by these results, regarding the manufacturing method according to the present invention, the selectivity is improved because of a highly active isomerization catalyst as compared with that in the manufacturing method by using the publicly known catalyst.

**[Table 2-3]**

| | | | | | | | | |
|---|---|---|---|---|---|---|---|---|
| Production of propylene at atmospheric pressure and 300°C without hydrogen | | | | | | | | |

| | Metathesis catalyst | Isomerization catalyst. | Butene conversion [%] | | Propylene selectivity [%] | | 2B/1B | |
|---|---|---|---|---|---|---|---|---|
| | | | 8h | 15h | 8h | 15h | 8h | 15h |
| Example 2-12 | WQ-15 | 1 | 35.3 | 32.8 | 80.4 | 79.7 | 3.9 | 3.7 |
| Example 2-13 | WQ-15 | 5 | 48.4 | 46.8 | 84.3 | 83.0 | 4.0 | 3.8 |
| Example 2-14 | WQ-15 | 7 | 52.9 | 50.8 | 85.3 | 83.8 | 3.9 | 3.7 |
| Comparative example 2-2 | WQ-15 | 12 | 34.2 | 28.5 | 66.6 | 63.5 | 0.8 | 0.7 |

### [Preparation of catalyst]

### (Preparation example 3-1) Preparation of metathesis catalyst (catalyst (a)) WQ-15

As in Preparation example 1-1 described above, WQ-15 was prepared.

### (Preparation example 3-2) Preparation of Isomerization catalyst (catalyst (b)) Y₂O₃ catalyst

An yttrium nitrate aqueous solution and 25% ammonia water were prepared and transferred to a flask containing water therein, followed by mixing. Here, the liquid transfer rate of the yttrium nitrate aqueous solution was kept constant, and the liquid transfer rate of ammonia water was controlled in such a way as to keep pH of the liquid in the flask at 8. Stirring was continued for 30 minutes after completion of the liquid transfer. Subsequently, the resulting precipitates were washed by repeating filtration and redispersion into water. The resulting wet cake was dried and, thereafter, was calcined in an air atmosphere at 550°C so as to obtain a white solid. This was made into a powder and, thereafter, compression molding was conducted. Pulverization and classification into the size of 150 to 500 µm were conducted so as to obtain a Y₂O₃ catalyst.

### (Preparation example 3-3) Preparation of catalyst (c) Al₂O₃ catalyst

Activated alumina NKHD-24 (produced by Sumitomo Chemical Co., Ltd., spherical formed body) was calcined in an air atmosphere at 500°C. Thereafter, pulverization and classification into the size of 150 to 500 µm were conducted, so as to obtain an Al₂O₃ catalyst. The specific surface area of the Al₂O₃ catalyst was 200 m²/g. In this regard, the specific surface area was measured with BELSORP-max-2 (produced by BEL Japan, Inc.).

### (Preparation example 3-4) Preparation of catalyst (c) Al₂O₃ catalyst (2)

Activated alumina NKHD-24 (produced by Sumitomo Chemical Co., Ltd., spherical formed body) was pulverized and classified into the size of 150 to 500 µm, so as to obtain an Al₂O₃ catalyst. The specific surface area of the Al₂O₃ catalyst was 160 m²/g. In this regard, the specific surface area was measured with BELSORP-max-2 (produced by BEL Japan, Inc.).

### (Example 3-1, Comparative examples 3-1 to 3-2)

### [Production 3-1 of propylene from ethylene and butene] (Reactor)

As for a reactor, the center of a stainless steel single pipe reactor was filled with physically mixed WQ-15 and Y₂O₃ catalyst or Al₂O₃ catalyst (the catalyst obtained from (Preparation example 3-3)) at a ratio shown in Table 3-1, and alumina balls serving as preheat retaining layers were filled on and under the catalysts.

### (Production of propylene)

The catalysts in the state of being filled in the reactor were subjected to a calcining treatment and a reduction treatment in advance and, furthermore, purging with nitrogen was conducted. Thereafter, the temperature of the catalyst bed was lowered to a reaction temperature. Subsequently, a mixed gas having the composition shown in Table 3-2 (prepared by mixing mixed butene produced by our company and high-purity ethylene) was supplied as raw material olefins so as to effect a flow reaction at a gauge pressure of 3.5 MPa. Here, the reaction temperature was specified to be 225°C, and the total feed rate of olefins was specified to be WHSV = 20 hr⁻¹ relative to the weight of WQ-15-Furthermore, hydrogen in an amount of 1 percent by volume of the total amount of supply of olefins and hydrogen was mixed with the raw material olefins and supplied to the reactor. Regarding a gas at a reactor outlet, the concentrations of ethylene, propylene, butenes, and pentenes were analyzed by using FID-GC so as to determine the butene conversion, the propylene selectivity, and the 2-butene/l-butene molar ratio (expressed as 2B/1B in Table and the drawing) of the outlet gas.

The time for which the butene conversion is at 65% or more from the start of the reaction is shown in Table 3-3. Furthermore, the progression of the ratio of 2-butene/1-butene is shown in Fig. 3-1.

As is clear from Table 3-3, regarding Example 3-1, in which the catalyst (a), the catalyst (b), and the catalyst (c) were used together, the butene conversion was maintained at 65% or more for a long term and, therefore, the activity of the metathesis catalyst was able to be maintained for a long term as compared with that in Comparative examples 3-1, in which the catalyst (c) was not used. In addition, as is clear from Fig. 3-1, regarding Example 3-1, in which the catalyst (a), the catalyst (b), and the catalyst (c) were used together, the 2-butene/1-butene molar ratio was maintained at high values for a long term and, therefore, deterioration of the activity of the isomerization catalyst was able to be suppressed as compared with that in Comparative example 3-2, in which the catalyst (b) was not used.

As is indicated by these results, regarding the manufacturing method, in which the catalyst (a), the catalyst (b), and the catalyst (c) were used together, deterioration of the activity of the metathesis catalyst and deterioration of the activity of the isomerization catalyst were suppressed at the same time as compared with that in the method, in which the catalyst (b) or the catalyst (c) was not used.

In this regard, in Fig. 3-1, the 2-butene/1-butene ratio in Comparative example 3-1 increases after 48 hours or more. This is because the butene conversion (the degree of progress of metathesis reaction, in which propylene is generated from butene and ethylene) is reduced and conversion of 2-butene, becomes difficult. Therefore, it is not indicated that the activity of the isomerization catalyst is improved (recovered) .

**[Table 3-1]**

| | | | |
|---|---|---|---|
| Composition ratio of catalyst layer (weight ratio) | | | |

| | WQ-15 | Y₂O₃ catalyst | Al₂O₃ catalyst |
|---|---|---|---|
| Example 3-1 | 1 | 2 | 2 |
| Comparative example 3-1 | 1 | 4 | 0 |
| Comparative example 3-2 | 1 | 0 | 4 |

**[Table 3-2]**

| | |
|---|---|
| Raw material composition of olefins | |

| Composition | Concentration percent by mole |
|---|---|
| Ethylene | 42.41 |
| 1-butene | 4.06 |
| 2-butene | 24.22 |
| Isobutene | 7.37 |
| n-butane | 15.31 |
| Isobutane | 5.97 |

In addition, very small amounts of ethane, propane, propylene, butadiene, 1-pentenes, 2-pentene, and water are contained.

**[Table 3-3]**

| | Time for which butene conversion is at 65% or more | Remarks |
|---|---|---|
| Example 3-1 | 100 hours or more | operation was stopped after 100 hours |
| Comparative example 3-1 | 48 hours | |
| Comparative example 3-2 | 100 hours or more | operation was stopped after 100 hours |

### (Examples 3-2 to 3-9, Comparative Examples 3-3 to 3-7)

### [Production 3-2 of propylene from ethylene and butene]

Propylene was produced from ethylene and butene in the same manner as in Example 3-1, except that the configuration of catalyst layer or reaction conditions were changed as indicated in Table 3-4.
The reaction results after 10 hours from the initiation of the reactions and time for butene conversion are shown in Table 3-5. The progression of 2-butene/1-butene ratio of a gas at a reactor outlet is shown in Figs. 3-2 to 3-5. The relationships between the 2-butene/1-butene ratio and the blend ratio of Y₂O₃ catalyst to Al₂O₃ catalyst after 10, 20, 40 hours from the initiation of the reactions are shown in Fig. 3-6. In the Tables 3-4 and 3-5, the results of Example 3-1 and Comparative Examples 3-1 and 3-2 are also indicated; and moreover, some examples are repeatedly indicated so as to facilitate the comparisons of the results.
From the comparison of Example 3-2, Comparative Example 3-3 and Comparative Example 3-4, it was demonstrated that the selectivity was improved without the addition of hydrogen and the effect of the third aspect of the present invention is employable.
From the comparison of Example 3-3, Comparative Example 3-5 and Comparative Example 3-6, it was demonstrated that when the catalyst containing an alkaline earth metal element was used as the catalyst (b) in the third aspect of the present invention, the effect of the present invention was achieved.
From the comparison of Examples 3-4 to 3-7 and Comparative Example 3-7, it was demonstrated that when the metal oxide catalyst containing at least two metal elements of at least one metal element selected from alkaline earth metal elements and at least one metal element selected from rare earth metals; or the metal oxide catalyst containing three components of at least one metal element (Component A) selected from alkaline earth metal elements, aluminum (Component B) and at least metal element (Component C) selected from rare earth metals, was used as the catalyst (b), the effect of the present invention was achieved.
Moreover, from Fig. 3-6, it was demonstrated that when the catalyst (b) and catalyst (c) were used in combination, the isomerization activity higher than a performance predicted from the blend ratio exhibits.

**[Table 3-4]**

| | | | | | | | | | | |
|---|---|---|---|---|---|---|---|---|---|---|
| Configuration of Catalyst Layer and Conditions of Flow Reactions | | | | | | | | | | |

| | Catalyst Layer | | | | | | Reaction conditions | | | |
|---|---|---|---|---|---|---|---|---|---|---|
| | Catalyst (a) | Catalyst (b) | Catalyst (c) | Weight Ratio | | | Temperature [°C] | Pressure [MPa·G] | H2 [%] | WHSV [hr⁻¹] |
| | | | | Catalyst (a) | Catalyst (b) | Catalyst (c) | | | | |
| Ex. 3-1 | WQ-15 | Y2O3 | Al2O3 | 1 | 2 | 2 | 225 | 3.5 | 1 | 20 |
| Comp.Ex. 3-1 | WQ-15 | Y2O3 | - | 1 | 4 | 0 | 225 | 3.5 | 1 | 20 |
| Cpmp.Ex. 3-2 | WQ-15 | - | Al2O3 | 1 | 0 | 4 | 225 | 3.5 | 1 | 20 |
| Ex. 3-2 | WQ-15 | Y203 | Al2O3 (2) | 1 | 2 | 2 | 300 | 3.5 | 0 | 40 |
| Comp.Ex. 3-3 | WQ-15 | Y2O3 | - | 1 | 4 | 0 | 300 | 3.5 | 0 | 40 |
| Comp.Ex. 3-4 | WQ-15 | - | Al2O3 (2) | 1 | 0 | 4 | 300 | 3.5 | 0 | 40 |
| Ex. 3-3 | WQ-15 | REF-1 | Al203 (2) | 1 | 2 | 2 | 225 | 3.5 | 1 | 40 |
| Comp.Ex. 3-5 | WQ-15 | REF-1 | - | 1 | 4 | 0 | 225 | 3.5 | 1 | 40 |
| Comp.Ex. 3-6 | WQ-15 | - | Al2O3 (2) | 1 | 0 | 4 | 225 | 3.5 | 1 | 40 |
| Ex. 3-4 | WQ-15 | ML-30 | Al2O3 | 1 | 2 | 2 | 225 | 3.5 | 1 | 40 |
| Ex.3-5 | WQ-15 | ML-30 | - | 1 | 4 | 0 | 225 | 3.5 | 1 | 40 |
| Comp.Ex. 3-7 | WQ-15 | - | Al2O3 | 1 | 0 | 4 | 225 | 3.5 | 1 | 40 |
| Ex. 3-6 | WQ-15 | Isomerization Catalyst 7 | Al2O3 | 1 | 2 | 2 | 225 | 3.5 | 1 | 40 |
| Ex. 3-7 | WQ-15 | Isomerization Catalyst 7 | - | 1 | 4 | 0 | 225 | 3.5 | 1 | 40 |
| Comp.Ex. 3-7 | WQ-15 | - | Al2O3 | 1 | 0 | 4 | 225 | 3.5 | 1 | 40 |
| Ex. 3-1 | WQ-15 | Y2O3 | Al2O3 | 1 | 2 | 2 | 225 | 3.5 | 1 | 20 |
| Ex. 3-8 | WQ-15 | Y203 | Al2O3 | 1 | 1 | 3 | 225 | 3.5 | 1 | 20 |
| Ex. 3-9 | WQ-15 | Y203 | Al2O3 | 1 | 0.5 | 3.5 | 225 | 3.5 | 1 | 20 |
| Comp.Ex. 3-1 | WQ-15 | Y2O3 | - | 1 | 4 | 0 | 225 | 3.5 | 1 | 20 |
| Comp.Ex. 3-2 | WQ-15 | - | Al2O3 | 1 | 0 | 4 | 225 | 3.5 | 1 | 20 |

In Table 3-4, "Al2O3" refers to the catalyst obtained in Preparation Example 3-3 and "Al2O3(2)" refers to the catalyst obtained in Preparation Example 3-4.

**[Table 3-5]**

| | | | | |
|---|---|---|---|---|
| Results of Flow Reactions | | | | |

| | Reaction Result after 10 hrs from Initiation of Reaction | | | Time for which conversion is 65% or more (hr) |
|---|---|---|---|---|
| | Butene Conversion (%) | Propylene Selectivity (%) | 2B/1B Mortar Ratio | |
| Ex. 3-1 | 68.9 | 92.1 | 5.9 | >100 |
| Comp.Ex. 3-1 | 69.1 | 91.8 | 5.9 | 60 |
| Comp.Ex. 3-2 | 68.2 | 88.0 | 3.3 | >100 |
| Ex. 3-2 | 67.7 | 87.3 | 4.0 | 15 |
| Comp.Ex 3-3 | 60.7 | 87.6 | 4.0 | 8 |
| Comp.Ex. 3-4 | 65.6 | 84.4 | 2.5 | 11 |
| Ex. 3-3 | 68.8 | 90.3 | 5.1 | 19 |
| Comp.Ex. 3-5 | 63.0 | 88.6 | 3.7 | 10 |
| Comp.Ex 3-6 | 68.3 | 85.7 | 2.7 | 38 |
| Ex. 3-4 | 68.3 | 91.5 | 5.9 | 21 |
| Ex. 3-5 | 67.9 | 91.7 | 5.8 | 12 |
| Camp.Ex. 3-7 | 68,3 | 86.6 | 2.6 | 37 |
| Ex. 3-6 | 68.6 | 91.2 | 5.6 | 24 |
| Ex. 3-7 | 68.9 | 91.8 | 5.9 | 17 |
| Comp.Ex. 3-7 | 68.3 | 86.6 | 2.6 | 37 |
| Ex. 3-1 | 68.9 | 92.1 | 5.9 | >100 |
| Ex. 3-8 | 69.3 | 90.7 | 5.8 | >100 |
| Ex. 3-9 | 68.8 | 91.3 | 5.3 | >100 |
| Comp.Ex. 3-1 | 69.1 | 91.8 | 5.9 | 60 |
| Comp.Ex. 3-2 | 68.2 | 88.0 | 3.3 | >100 |

### [Industrial Applicability]

The present invention can be used as a manufacturing method, in which mutual conversion of an olefin serving as a raw material to other olefin occurs.

## Claims

1. A method for manufacturing an olefin through reaction between the same type of or different types of raw material olefins to obtain an olefin having a structure different from the structure of the raw material olefins, the method comprising using:
a catalyst containing at least one type of metal element selected from the group consisting of tungsten, molybdenum, and rhenium; and
a metal oxide catalyst containing at least two types of metal elements including at least one type of metal element selected from alkaline earth metal elements and at least one type of metal element selected from rare earth elements.

2. A method for manufacturing an olefin through reaction between the same type of or different types of raw material olefins to obtain an olefin having a structure different from the structure of the raw material olefins, the method comprising using:
a catalyst containing at least one type of metal element selected from the group consisting of tungsten, molybdenum, and rhenium; and
a metal oxide catalyst containing three components including at least one type of metal element (component A) selected from alkaline earth metal elements, aluminium (component B), and at least one type of metal element (component C) selected from rare earth elements.

3. A method for manufacturing an olefin through reaction between the same type of or different types of raw material olefins to obtain an olefin having a structure different from the structure of the raw material olefins, the method comprising using:
a catalyst (a) containing at least one type of metal element selected from the group consisting of tungsten, molybdenum, and rhenium;
a catalyst (b) containing at least one type of metal element selected from alkaline earth metal elements and rare earth elements; and
a catalyst (c) containing alumina.

4. The method for manufacturing an olefin according to Claim 1 comprising the steps of:
obtaining an olefin from the same type or different types of olefins among raw material olefins including an olefin having the carbon number of 3 or more, the resulting olefin being different from the same type or the different types of olefins, in a reactor containing a metathesis catalyst, as a metathesis reaction step; and
isomerizing an olefin to shift the position of a double bond within the molecule in a reactor which contains an isomerization catalyst and in which raw material olefines are present which are the same as or different from the raw material olefins and include an olefin having the carbon number of 3 or more,
wherein the metathesis catalyst is a catalyst containing at least one type of metal element selected from the group consisting of tungsten, molybdenum, and rhenium, and the isomerization catalyst is a metal oxide catalyst containing at least two types of metal elements including at least one type of metal element selected from alkaline earth metal elements and at least one type of metal element selected from rare earth elements.

5. The method for manufacturing an olefin according to Claim 2 comprising the steps of:
obtaining an olefins from the same type or different types of olefins among raw material olefins including an olefin having the carbon number of 3 or more, the resulting olefin being different from the same type or the different types of olefins, in a reactor containing a metathesis catalyst, as a metathesis reaction step; and
isomerizing an olefin to shift the position of a double bond within the molecule in a reactor which contains an isomerization catalyst and in which raw material olefins are present which are the same as or different from the raw material olefins and include an olefin having the carbon number of 3 or more,
wherein the metathesis catalyst is
a catalyst containing at least one type of metal element
selected from the group consisting of tungsten, molybdenum, and rhenium, and
the isomerization catalyst is a metal oxide catalyst containing the following three components including
at least one type of metal element (component A) selected from alkaline earth metal elements,
aluminum (component B), and
at least one type of metal element (component C) selected from rare earth elements.

6. The method for manufacturing an olefin according to Claim 3 comprising the steps of:
obtaining an olefin from the same type or different types of olefins among raw material olefins including an olefin having the carbon number of 3 or more, the resulting olefin being different from the same type or the different types of olefins, in a reactor containing a metathesis catalyst, as a metathesis reaction step; and
isomerizing an olefin to shift the position of a double bond within the molecule in a reactor which contains an isomerization catalyst and in which raw material olefins are present which are the same as or different from the raw material olefins and include an olefin having the carbon number of 3 or more,
wherein the metathesis catalyst is a catalyst (a) containing at least one type of metal element selected from the group consisting of tungsten, molybdenum, and rhenium,
the isomerization catalyst is a metal oxide catalyst (b) containing at least one type of metal element selected from alkaline earth metal elements and rare earth elements, and
a catalyst (c) containing alumina is used together with the metathesis catalyst and/or the isomerization catalyst.

7. The method for manufacturing an olefin according to any one of Claims 4 to 6, **characterized in that** the isomerization reaction step and the metathesis reaction step are conducted in one reactor.

8. The method for manufacturing an olefin according to any one of Claims 4 to 6, **characterized in that** the isomerization reaction step and the metathesis reaction step are conducted in separate reactors, and the olefin generated in the isomerization reaction step is used as at least a part of the raw material olefin for the metathesis reaction step.

9. The method for manufacturing an olefin according to any one of Claims 1 to 3, **characterized in that** hydrogen is also supplied together with the raw material olefin.

10. The method for manufacturing an olefin according to any one of Claims 4 to 8, **characterized in that** hydrogen is also supplied together with the raw material in at least one of the isomerization reaction step and the metathesis reaction step.

11. The method for manufacturing an olefin according to any one of Claims 4 to 8, wherein the raw material olefin comprising an olefin having the carbon number of 3 or more comprises n-butene.

12. The method for manufacturing an olefin according to any one of Claims 1 to 11, wherein the raw material olefin comprises ethylene.

13. The method for manufacturing an olefin according to any one of Claims 1 to 12, **characterized in that** the metal element selected from the alkaline earth metal elements is magnesium or calcium.

14. The method for manufacturing an olefin according to any one of Claims 1 to 13, **characterized in that** the metal element selected from the rare earth elements is yttrium or lanthanum.

15. The method for manufacturing an olefin according to Claim 3 or Claim 6, **characterized in that** the catalyst (c) is a catalyst having a specific surface area of 50 m²/g or more.

16. The method for manufacturing an olefin according to Claim 3 or Claim 6, **characterized in that** the catalyst (a), the catalyst (b), and the catalyst (c) are formed bodies made independently, and the individual formed bodies are present together in one reactor.

17. The method for manufacturing an olefin according to Claim 3 or Claim 6, **characterized in that** the catalyst (a) is a formed body (i) made independently, the catalyst (b) and the catalyst (c) constitute a formed body (ii) by being mixed with each other, and the formed body (i) and the formed body (ii) are present together in one reactor.

18. The method for manufacturing an olefin according to Claim 2 or Claim 5, wherein the content of the component A in the isomerization catalyst is 80% or less on a mole fraction basis, where a total of metal elements of the components A, B, and C is assumed to be 100%.

19. The method for manufacturing an olefin according to Claim 2 or Claim 5, wherein the content of the component B in the isomerization catalyst is 50% or less on a mole fraction basis, where a total of metal elements of the components A, B, and C is assumed to be 100%.

20. The method for manufacturing an olefin according to Claim 2 or Claim 5, wherein the content of the component C in the isomerization catalyst is 5% or more on a mole fraction basis, where a total of metal elements of the components A, B, and C is assumed to be 100%.

21. An isomerization catalyst for producing an olefin comprising a metal oxide containing three components including at least one type of metal element (component A) selected from alkaline earth metal elements, aluminum (component B), and at least one type of metal element (component C) selected from rare earth elements.

22. The isomerization catalyst according to Claim 21, wherein the content of the component A is 80% or less on a mole fraction basis, where a total of metal elements of the components A, B, and C is assumed to be 100%.

23. The isomerization catalyst according to Claim 21 or Claim 22, wherein the content of the component B is 50% or less on a mole fraction basis, where a total of metal elements of the components A, B, and C is assumed to be 100%.

24. The isomerization catalyst according to any one of Claims 21 to 23, wherein the content of the component C is 5% or more on a mole fraction basis, where a total of metal elements of the components A, B, and C is assumed to be 100%.

25. The isomerization catalyst according to any one of Claims 21 to 24, wherein the specific surface area is 10 m²/g or more.

26. The isomerization catalyst according to any one of Claims 21 to 25, **characterized in that**
the isomerization catalyst is produced by calcining a catalyst precursor,
the catalyst precursor comprises
at least one type of metal element (component A) selected from alkaline earth metal elements,
aluminum (component B), and
at least one type of metal element (component C) selected from rare earth elements,
wherein at least a part thereof has a layered double hydroxide (LDH) structure.

27. A catalyst precursor of an isomerization catalyst for producing an olefin, the catalyst precursor **characterized by**
comprising at least one type of metal element (component A) selected from alkaline earth metal elements,
aluminum (component B), and
at least one type of metal element (component C) selected from rare earth elements,
wherein 30% to 80% of component A, 9% to 50% of component B, and 5% to 50% of component C are contained on a mole fraction basis, where a total of metal elements of the components A, B, and C is assumed to be 100%, and at least a part thereof has a layered double hydroxide (LDH) structure.
